# EUROPEAN PATENT APPLICATION

(11) **EP 4 585 225 A1**
(43) Date of publication of application: **16.07.2025**
(21) Application number: 23863259.0
(22) Date of filing: 08.09.2023
(51) Int. Cl.: A61K 45/00, A61P 19/00, C12N 5/0797, C12N 5/10, C12N 15/12, A61K 35/545, A61P 25/00, A61K 38/16, C07K 14/475

(54) **SPINAL CORD INJURY THERAPEUTIC TARGETING FROM ACUTE PHASE TO SUBACUTE PHASE**

(30) Priority: 08.09.2022 JP 2022143261
(71) Applicant: Keio University, Tokyo, 108-8345 (JP); Kringle Pharma, Inc., Ibaraki-shi, Osaka 567-0085 (JP)
(72) Inventor: SUEMATSU Yu, Tokyo 160-8582 (JP); NAGOSHI Narihito, Tokyo 160-8582 (JP); NAKAMURA Masaya, Tokyo 160-8582 (JP); OKANO Hideyuki, Tokyo 160-8582 (JP)
(74) Representative: Krauss, Jan
(86) International application number: PCT/JP2023/032768
(87) International publication number: WO 2024/053721

(57) **Abstract**

[Problem] To provide a therapeutic for spinal cord injury, especially severe spinal cord injury, from the acute phase to the subacute phase, the conventional treatment of which has been difficult. [Solution] A therapeutic for spinal cord injury from the acute phase to the subacute phase, the therapeutic containing the following: 1) (a) a hepatocyte growth factor protein or a substance having a c-Met phosphorylation effect equivalent to that of a hepatocyte growth factor protein or (b) a gene encoding a hepatocyte growth factor protein or a gene encoding a substance having a c-Met phosphorylation effect equivalent to that of a hepatocyte growth factor protein and 2) pluripotent stem cells.

## Description

### Technical Field

The present disclosure relates to a therapeutic agent for spinal cord injury from an acute phase to a subacute phase, in particular, a therapeutic agent for severe spinal cord injury. The present disclosure also relates to a motor dysfunction-alleviating agent for spinal cord injury from an acute phase to a subacute phase (in particular, a lower limb motor function improver, a walking ability improver, or a walking ability-acquiring agent), an inhibitor for spinal cord cavitation in a lesion site of a spinal cord, an inhibitor for atrophy of an injured spinal cord, an inhibitor for demyelination after spinal cord injury, an improver for remyelination after spinal cord injury, a promoter for migration/axon elongation in a lesion site of a spinal cord, and a method of increasing a survival and engraftment rate of pluripotent stem cells for transplantation.

The respective agents of the present disclosure are sometimes collectively referred to as "agents of the present disclosure."

The present application claims priority from Japanese Patent Application No. 2022-143261, the disclosure of which is incorporated herein by reference.

### Background Art

### (Spinal Cord Injury)

Spinal cord injury is a disease state showing the paralysis of sensory, motor, and autonomic nervous systems below a lesion with the injury of a spinal cord parenchyma by a trauma or the like as a trigger, and the total number of the patients of the injury in Japan has reached 150,000 or more. Further, about 5,000 new patients are occurring every year.

However, it has been considered that the central nervous system of a mammal is not regenerated once the system is injured, and hence under the current circumstances, no effective therapeutic method has been established yet.

### (Hepatocyte Growth Factor)

A hepatocyte growth factor (hereinafter sometimes abbreviated as "HGF" in this description) protein was discovered as a bioactive protein having growth-promoting activity on a mature hepatocyte. As a result of subsequent investigations, it has been known that the HGF protein acts on the c-Met receptors of many cells, such as an epithelial cell and a vascular endothelial cell, as well as a hepatocyte, to be involved in the damage repair and regeneration of tissues and organs. The HGF protein can be produced in a large amount as a recombinant protein by a bioengineering approach, and the recombinant HGF protein has been expected to be applied as a therapeutic agent not only for hepatitis and liver cirrhosis but also for a renal disease, a wound, or the like.

Meanwhile, many investigations in the expression and functional analysis of a gene including a recent knock-out/knock-in mouse approach have revealed that the HGF protein also has promoting actions on the survival of a nerve cell and the elongation of a neurite, and is hence a factor important as a neurotrophic factor.

The HGF protein shows neurotrophic factor activity on each of nerve cells, such as a hippocampal neuron, a dopaminergic neuron, a cerebellar granule cell, a sensory neuron, and a motor neuron. The HGF protein shows a strong survival-promoting action particularly on the motor neuron, and the activity is comparable to the activity of a glial cell line-derived neurotrophic factor (GDNF) known to have the strongest survival-promoting action on the motor neuron.

It has been reported that the HGF protein may be used as a therapeutic agent for various nerve diseases including amyotrophic lateral sclerosis (ALS) and spinal cord injury on the basis of such neurotrophic activity (see Patent Literature 1).

### (Prior Non Patent Literature)

In Non Patent Literature 1, there is a disclosure "that the administration of a hepatocyte growth factor and an iPS cell-derived NSC after spinal cord injury is assumed to be effective in regeneration after the spinal cord injury." However, there is no disclosure or suggestion of the configuration of each of the agents of the present disclosure.

In Non Patent Literature 2, there is a disclosure of a "therapeutic method including applying a gelatin-furfurylamine (FA) hydrogel and a CBD-HGF or a HGF in combination to an animal whose spinal cord has been injured by compression." However, there is no disclosure or suggestion of the configuration of each of the agents of the present disclosure.

In Patent Literature 2, there is a disclosure of a "columnar HGF protein-containing sustained release preparation obtained by mixing an aqueous solution of a HGF protein and a solution of atelocollagen in a phosphate buffer, then freeze-drying the mixture, and subjecting the dried product to compression molding." However, there is no disclosure or suggestion of the configuration of each of the agents of the present disclosure.

In Patent Literature 3, there is a disclosure of a "therapeutic agent for nerve injury including a differentiated cell-derived pluripotent stem cells." However, there is no disclosure or suggestion of the configuration of each of the agents of the present disclosure.

### Citation List

### Patent Literature

[PTL 1] JP 2018-44000 A
[PTL 2] JP 5419045 B2
[PTL 3] JP 2009-215191 A

### Non Patent Literature

[NPL 1] Int. J. Mol. Sci. 2019, 20(15), 3838
[NPL 2] Sci. Rep. 2018, 8: 917.

### Summary of Invention

### Technical Problem

An object of the present disclosure is to provide a therapeutic agent for spinal cord injury from an acute phase to a subacute phase that has heretofore been difficult to treat, in particular, severe spinal cord injury. Another object of the present disclosure is to provide a therapeutic agent applicable even to severe spinal cord injury on which a sufficient effect is not obtained with a HGF alone or an iPS alone, for example, as follows: load walking cannot be performed.

The inventors of the present disclosure have recognized that an agent including the following (1) and (2) has a therapeutic effect and a motor dysfunction-alleviating effect on severe spinal cord injury from an acute phase to a subacute phase, in particular, severe spinal cord injury on which no effect is obtained with a HGF alone or an iPS alone, and thus the inventors have completed the present disclosure:
(1) a HGF protein; and
(2) an iPS cell-derived neural stem cell and/or neural progenitor cells.

The present disclosure includes the following.
1. A therapeutic agent for spinal cord injury from an acute phase to a subacute phase, including the following:
   (1) (a) a hepatocyte growth factor protein or a substance having a c-Met phosphorylation action comparable to that of the hepatocyte growth factor protein, or (b) a gene encoding the hepatocyte growth factor protein or a gene encoding the substance having a c-Met phosphorylation action comparable to that of the hepatocyte growth factor protein; and
   (2) a pluripotent stem cell.
2. The therapeutic agent according to Item 1, wherein the pluripotent stem cells are an iPS cell-derived neural stem cell and/or neural progenitor cells.
3. The therapeutic agent according to Item 1, wherein the spinal cord injury is severe spinal cord injury.
4. The therapeutic agent according to Item 1, wherein the substance having a c-Met phosphorylation action is a hepatocyte growth factor protein, the pluripotent stem cells are an iPS cell-derived neural stem cell and/or neural progenitor cells, and the spinal cord injury from the acute phase to the subacute phase is severe spinal cord injury.
5. The therapeutic agent according to Item 4, wherein the hepatocyte growth factor protein is intrathecally administered.
6. The therapeutic agent according to Item 5, wherein the hepatocyte growth factor protein is administered into a subarachnoid cavity.
7. The therapeutic agent according to any one of Items 1 to 6, wherein the treatment is promotion of regeneration of an injured spinal cord and/or alleviation of an accessory symptom accompanying the spinal cord injury.
8. The therapeutic agent according to Item 7, wherein the promotion of the regeneration of the injured spinal cord is promotion of axon extension, inhibition of atrophy of the spinal cord, an increase in survival and engraftment rate of the pluripotent stem cells, inhibition of demyelination, an improvement in remyelination, and/or inhibition of spinal cord cavitation.
9. The therapeutic agent according to Item 7, wherein the promotion of the regeneration of the injured spinal cord is activation of an endogenous nerve cell, reconstruction of a neural network, and/or promotion or encouragement of an increase in number of functional nerve fibers.
10. The therapeutic agent according to Item 7, wherein the accessory symptom accompanying the injured spinal cord is motor dysfunction.
11. The therapeutic agent according to Item 10, wherein the motor dysfunction is motor dysfunction of a lower limb.
12. The therapeutic agent according to Item 11, wherein the motor dysfunction is walking ability loss.
13. A therapeutic agent for spinal cord injury from an acute phase to a subacute phase, including a hepatocyte growth factor protein,
   wherein the hepatocyte growth factor protein is supplied to a lesion site of a spinal cord by intrathecal administration, and an iPS cell-derived neural stem cell and/or neural progenitor cells are administered to the lesion site of the spinal cord.
14. A therapeutic agent for spinal cord injury from an acute phase to a subacute phase, including an iPS cell-derived neural stem cell and/or neural progenitor cells,
   wherein the iPS cell-derived neural stem cell and/or neural progenitor cells are administered to a lesion site of a spinal cord during or after supply of a hepatocyte growth factor protein to the lesion site of the spinal cord by intrathecal administration.
15. A motor dysfunction-alleviating agent for spinal cord injury from an acute phase to a subacute phase, including the following:
   (1) a hepatocyte growth factor protein; and
   (2) an iPS cell-derived neural stem cell and/or neural progenitor cells,
   wherein the hepatocyte growth factor protein is supplied to a lesion site of a spinal cord by intrathecal administration, and the iPS cell-derived neural stem cell and/or neural progenitor cells are administered to the lesion site of the spinal cord.
16. The motor dysfunction-alleviating agent according to Item 15, wherein the motor function is a lower limb motor function or a walking function.
17. An inhibitor for demyelination after spinal cord injury for spinal cord injury from an acute phase to a subacute phase, including the following:
   (1) a hepatocyte growth factor protein; and
   (2) an iPS cell-derived neural stem cell and/or neural progenitor cells,
   wherein the hepatocyte growth factor protein is supplied to a lesion site of a spinal cord by intrathecal administration, and the iPS cell-derived neural stem cell and/or neural progenitor cells are administered to the lesion site of the spinal cord.
18. An improver for remyelination after spinal cord injury for spinal cord injury from an acute phase to a subacute phase, including the following:
   (1) a hepatocyte growth factor protein; and
   (2) an iPS cell-derived neural stem cell and/or neural progenitor cells,
   wherein the hepatocyte growth factor protein is supplied to a lesion site of a spinal cord by intrathecal administration, and the iPS cell-derived neural stem cell and/or neural progenitor cells are administered to the lesion site of the spinal cord.
19. An inhibitor for spinal cord cavitation for spinal cord injury from an acute phase to a subacute phase, including the following:
   (1) a hepatocyte growth factor protein; and
   (2) an iPS cell-derived neural stem cell and/or neural progenitor cells,
   wherein the hepatocyte growth factor protein is supplied to a lesion site of a spinal cord by intrathecal administration, and the iPS cell-derived neural stem cell and/or neural progenitor cells are administered to the lesion site of the spinal cord.
20. An inhibitor for atrophy of a spinal cord for spinal cord injury from an acute phase to a subacute phase, including the following:
   (1) a hepatocyte growth factor protein; and
   (2) an iPS cell-derived neural stem cell and/or neural progenitor cells,
   wherein the hepatocyte growth factor protein is supplied to a lesion site of a spinal cord by intrathecal administration, and the iPS cell-derived neural stem cell and/or neural progenitor cells are administered to the lesion site of the spinal cord.
21. A promoter for axon elongation in a lesion site for spinal cord injury from an acute phase to a subacute phase, including the following:
   (1) a hepatocyte growth factor protein; and
   (2) an iPS cell-derived neural stem cell and/or neural progenitor cells,
   wherein the hepatocyte growth factor protein is supplied to a lesion site of a spinal cord by intrathecal administration, and the iPS cell-derived neural stem cell and/or neural progenitor cells are administered to the lesion site of the spinal cord.
22. A promoter for reconstruction of a neural network of a lesion site and/or an increase in number of functional never fibers thereof for spinal cord injury from an acute phase to a subacute phase, including the following:
   (1) a hepatocyte growth factor protein; and
   (2) an iPS cell-derived neural stem cell and/or neural progenitor cells,
   wherein the hepatocyte growth factor protein is supplied to a lesion site of a spinal cord by intrathecal administration, and the iPS cell-derived neural stem cell and/or neural progenitor cells are administered to the lesion site of the spinal cord.
23. A therapeutic method for spinal cord injury from an acute phase to a subacute phase, including the following steps:
   (1) a step of supplying (a) a hepatocyte growth factor protein or a substance having a c-Met phosphorylation action comparable to that of the hepatocyte growth factor protein, or (b) a gene encoding the hepatocyte growth factor protein or a gene encoding the substance having a c-Met phosphorylation action comparable to that of the hepatocyte growth factor protein to a lesion site of a spinal cord by intrathecal administration or intraspinal administration; and
   (2) a step of administering a pluripotent stem cell to the lesion site of the spinal cord.
24. A kit for treating spinal cord injury from an acute phase to a subacute phase, including the following:
   (1) (a) a hepatocyte growth factor protein or a substance having a c-Met phosphorylation action comparable to that of the hepatocyte growth factor protein, or (b) a gene encoding the hepatocyte growth factor protein or a gene encoding the substance having a c-Met phosphorylation action comparable to that of the hepatocyte growth factor protein; and
   (2) a pluripotent stem cell,
   wherein the hepatocyte growth factor protein or the substance having a c-Met phosphorylation action comparable to that of the hepatocyte growth factor protein, or the gene encoding the hepatocyte growth factor protein or the gene encoding the substance having a c-Met phosphorylation action comparable to that of the hepatocyte growth factor protein is supplied to a lesion site of a spinal cord by intrathecal administration or intraspinal administration, and the pluripotent stem cells are administered to the lesion site of the spinal cord.
25. A method of increasing a survival and engraftment rate of pluripotent stem cells for transplantation, including bringing a hepatocyte growth factor into contact with the pluripotent stem cells for transplantation.

### Advantageous Effects of Invention

The agents of the present disclosure each have one or more of the following effects:
(1) an alleviating effect on motor dysfunction due to spinal cord injury (a recovery effect on a lower limb motor function or a walking ability-acquiring effect);
(2) an inhibiting effect on spinal cord cavitation in a lesion site of a spinal cord;
(3) an inhibiting effect on the atrophy of an injured spinal cord;
(4) a migration/axon elongation effect in the lesion site of the spinal cord;
(5) an increasing effect on the survival and engraftment rate of transplanted cells;
(6) an inhibiting effect on demyelination after the spinal cord injury;
(7) an improving effect on remyelination after the spinal cord injury; and
(8) a reinforcing effect on neurotransmission in the lesion site of the spinal cord.

### Brief Description of Drawings

FIG. 1 is a diagram for illustrating the outline of the production of a spinal cord injury model.
FIG. 2 is a graph for showing the evaluation of a motor function based on a BBB score.
FIG. 3 is a set of graphs for showing the evaluation of the motor function based on a lower limb stride length.
FIG. 4 is a set of photographs for showing the evaluation of the motor function by the recognition of a walking ability.
FIG. 5 is a set of photographs for showing the result of the HE staining of a collected spinal cord tissue and the result of the HNA fluorescent staining thereof (HNA fluorescent staining in the lowermost figure is the result of a combination group).
FIG. 6 is a graph for showing the result of the measurement of the area of a spinal cord area.
FIG. 7 is a set of photographs for showing the result of the STEM121 fluorescent staining of the collected spinal cord tissue.
FIG. 8 is a set of photographs for showing the results of the detection of a HNA positive cell, an APC positive cell, a GFAP positive cell, a HuC/D positive cell, and a Ki-67 positive cell.
FIG. 9 is a set of graphs for showing the results of the measurement of the percentage of the Ki-67 positive cells (%) and the percentage of Nestin positive cells (%).
FIG. 10 is a graph for showing the evaluation of a serotonergic neuron around a lesion epicenter.
FIG. 11 is a set of photographs for showing the result of the myelin gold black staining of the collected spinal cord tissue.
FIG. 12 is a graph for showing the result of the measurement of a myelin sheath area around the lesion epicenter.
FIG. 13 is a diagram for illustrating the outline of a method of measuring the survival and engraftment rate of transplanted cells.
FIG. 14 is a set of photographs for showing the evaluation of the fluorescence properties of the transplanted cells by IVIS.
FIG. 15 is a graph for showing the result of the measurement of the survival and engraftment rate of the transplanted cells.
FIG. 16 is a diagram for illustrating the outline of a mechanism for the therapeutic effect of a therapeutic agent of the present disclosure.
FIG. 17 is a set of diagrams, photographs, and graphs for showing the result of gait analysis by kinematics and the result of electrophysiological analysis with a MEP.
FIG. 18 is a graph for showing the evaluation of a regenerated nerve fiber (NF-H) around the lesion epicenter.
FIG. 19 is a graph for showing the evaluation of the motor function based on the BBB score in which the number of cases is increased.
FIG. 20 is a graph for showing the evaluation of the motor function based on the lower limb stride length in which the number of cases is increased.

### Description of Embodiments

### (Target of the Present Disclosure)

The targets of the present disclosure (agents of the present disclosure) each relate to a therapeutic agent for spinal cord injury from an acute phase to a subacute phase, in particular, a therapeutic agent for severe spinal cord injury. Above all, the agents may each be a therapeutic agent for severe spinal cord injury on which no effect is obtained with a HGF alone or an iPS alone. The present disclosure also relates to a motor dysfunction-alleviating agent for spinal cord injury from an acute phase to a subacute phase (in particular, a therapeutic agent for severe spinal cord injury, above all, severe spinal cord injury on which no effect is obtained with a HGF alone or an iPS alone) (in particular, a walking ability improver or a walking ability-acquiring agent), an inhibitor for spinal cord cavitation in a lesion site of a spinal cord, an inhibitor for atrophy of an injured spinal cord, and a promoter for migration/axon elongation in a lesion site of a spinal cord.

The agents of the present disclosure each include the following:
(1) (a) a HGF protein or a substance having a c-Met phosphorylation action comparable to that of the HGF protein, or (b) a gene encoding the HGF protein or a gene encoding the substance having a c-Met phosphorylation action comparable to that of the HGF protein;
(2) a pluripotent stem cell.

Each of the above-mentioned (a) and (b) is preferably intended for intrathecal administration or intraspinal administration. More specifically, it is more preferred that the above-mentioned (a) be intended for intrathecal administration, and the above-mentioned (b) be intended for intraspinal administration.

The present disclosure is also directed to the use of the above-mentioned (1) and/or the above-mentioned (2) in the production of a therapeutic agent for spinal cord injury, a motor dysfunction-alleviating agent, an inhibitor for demyelination after spinal cord injury, an improver for remyelination after spinal cord injury, an inhibitor for spinal cord cavitation, an inhibitor for atrophy of a spinal cord, a promoter for axon extension in a lesion site, and a promoter for reconstruction of a neural network and/or an increase in number of functional never fibers.

### (Spinal Cord Injury)

Although phases ranging from the acute phase to subacute phase of spinal cord injury may each serve as a target to be treated with each of the agents of the present disclosure, the agent may be preferably intended for the treatment of severe spinal cord injury that has been difficult to treat (in particular, the acquisition of a walking ability has been difficult) by a related-art therapeutic method.

The term "severe" means a patient who cannot perform lower limb loading.

The treatment includes an improvement, alleviation, relapse prevention, and complete recovery.

### (Target to be treated)

The targets to be treated with the agents of the present disclosure are not particularly limited as long as the targets each have a spinal cord. Examples thereof may include a human, a monkey, a cow, a horse, a pig, a sheep, a dog, a cat, a rat, a mouse, a rabbit, a hamster, a guinea pig, and a chimpanzee.

### (HGF Protein and Gene encoding HGF Protein)

The kind of the HGF protein in the present disclosure is not particularly limited, and for example, HGF proteins derived from various animals (natural HGF proteins or recombinant proteins produced by a genetic engineering technology) may each be suitably used. In the present disclosure, for example, a HGF protein derived from an animal to which each of the agents of the present disclosure is applied is preferably used. For example, when each of the agents of the present disclosure is applied to a human, a human-derived HGF protein (hereinafter sometimes referred to as "human HGF protein") is suitably used as the HGF protein to be used in the present disclosure.

A recombinant human HGF protein is more preferred.

In addition, the HGF protein to be used in the present disclosure may be a deletion type (dHGF) from which 5 amino acid residues have been deleted.

The human HGF protein is preferably, for example, a protein to be encoded by DNA formed of a base sequence represented by SEQ ID NO: 1 or SEQ ID NO: 2. More specifically, for example, a protein formed of an amino acid sequence represented by SEQ ID NO: 3, a protein formed of an amino acid sequence represented by SEQ ID NO: 4, a protein formed of an amino acid sequence represented by SEQ ID NO: 5, or a protein formed of an amino acid sequence represented by SEQ ID NO: 6 is preferred.

Of those, a protein having an amino acid sequence represented by SEQ ID NO: 5 or SEQ ID NO: 6 is preferred as the human HGF protein, and a protein formed of an amino acid sequence represented by SEQ ID NO: 5 or SEQ ID NO: 6 is more preferred. For example, a HGF protein formed of the amino acid sequence represented by SEQ ID NO: 6 is a 5-amino acid-deficient HGF protein (dHGF) from which 5 amino acid residues, that is, the 131st to 135th amino acid residues of the amino acid sequence represented by SEQ ID NO: 5 have been deleted. Both the proteins each having an amino acid sequence represented by SEQ ID NO: 5 or SEQ ID NO: 6 are HGF proteins (natural HGF proteins) naturally present in a human body, and each have, for example, mitogen activity or motogen activity as a HGF protein.

The HGF protein to be used in the present disclosure includes a protein having a sequence identity of at least about 80% or more with each of the amino acid sequences of HGF proteins (natural HGF proteins) derived from various animals, preferably a protein having a sequence identity of about 90% or more therewith, more preferably a protein having a sequence identity of about 95% or more therewith, the protein having bioactivities (mitogen activity and motogen activity) as a HGF protein. With regard to the amino acid sequences, the term "sequence identity" means consistency in sequence-forming amino acid residue between the sequences of proteins when the primary structures of the proteins are compared to each other, and the term "% or more" means the degree of the consistency.

The fact that the HGF protein has the mitogen activity and the motogen activity described above may be recognized in accordance with, for example, a method described in J. Biol. Chem. 273, 22913-22920, 1998. A protein whose mitogen activity and motogen activity measured in accordance with J. Biol. Chem. 273, 22913-22920, 1998 described above are each typically about 50% or more, preferably about 70% or more, more preferably about 80% or more, still more preferably about 90% or more of that of each of the natural HGF proteins is preferably used.

A protein having the above-mentioned sequence identity with each of the natural HGF proteins is, for example, a protein including an amino acid sequence, which is obtained by substituting, deleting, and/or inserting one to several amino acid residues of, from, and/or into an amino acid sequence represented by SEQ ID NO: 5 or 6, or an amino acid sequence, which is obtained by modifying one to several amino acid residues thereof, the protein having bioactivity as a HGF protein.

The term "several" typically means 1 to 8 (1, 2, 3, 4, 5, 6, 7, or 8), and typically means 8, preferably 6, more preferably 5, still more preferably 3, particularly preferably 2. An amino acid to be inserted or an amino acid to be substituted is preferably a natural amino acid, but may be a non-natural amino acid except 20 kinds of amino acids to be encoded by genes. Although the non-natural amino acid may be any compound as long as the compound has an amino group and a carboxyl group, the acid is, for example, γ-aminobutyric acid.

The substitution of an amino acid residue means the substitution of an amino acid residue in polypeptide with another amino acid residue, and is preferably conservative substitution. The term "conservative substitution" means the substitution of one to several amino acid residues in the polypeptide with other chemically similar amino acid residues so that the activity of the polypeptide may remain substantially unchanged. Such case is, for example, a case in which a hydrophobic amino acid residue is substituted with another hydrophobic amino acid residue, or a case in which a polar amino acid residue is substituted with another polar amino acid residue having the same charge. A functionally similar amino acid that can perform such substitution is known in the art for each amino acid. Examples of the amino acid include: amino acids each having a non-polar (hydrophobic) side chain, such as glycine, alanine, valine, isoleucine, leucine, proline, tryptophan, phenylalanine, and methionine; neutral amino acids each having a polar side chain, such as serine, threonine, tyrosine, glutamine, asparagine, and cysteine; (basic) amino acids each having a positive charge, such as arginine, histidine, and lysine; and (acidic) amino acids each having a negative charge, such as aspartic acid and glutamic acid.

The agents of the present disclosure may each include only one kind of HGF protein, or may each include two or more kinds of the above-mentioned HGF proteins.

Proteins prepared by various methods may each be used as the HGF protein to be used in each of the agents of the present disclosure as long as the proteins are each purified to such an extent as to be usable as a medicine. Various methods have been known as methods of preparing the HGF protein, and the protein may be obtained by, for example, extracting a protein from an organ, such as a liver, a spleen, a lung, bone marrow, a brain, a kidney, or a placenta, of a mammalian animal, such as a rat, a cow, a horse, or a sheep, or a hemocyte, such as a thrombocyte or a leukocyte, plasma, or serum of the animal, and purifying the extract.

To extract the HGF protein from the above-mentioned biotissue or the like, and to purify the protein, for example, the following method may be adopted: carbon tetrachloride is intraperitoneally administered to a rat to bring the rat into a hepatitis state; the liver of the rat is removed and pulverized; and the resultant protein is purified by a typical protein purification method, such as column chromatography or HPLC with S-sepharose or heparin sepharose.

In addition, the HGF protein may be obtained by culturing a primary culture cell or an established cell that produces the HGF protein; and separating the protein from the culture (e.g., a culture supernatant or a culture cell), followed by its purification. Alternatively, a target recombinant HGF protein may be obtained as follows: a gene encoding the HGF protein (preferably DNA formed of a base sequence represented by SEQ ID NO: 1 or 2) is incorporated into an appropriate vector by a genetic engineering approach; the resultant is inserted into a proper host to be subjected to transformation; and the protein is obtained from the culture of the transformant (see, for example, Biochem. Biophys. Res. Commun. 180: 1151-1158, 1991; J. Clin. Invest. 87: 1853-1857, 1991; or Protein Expr. Purif. 70: 231-235, 2010).

The above-mentioned host cells are not particularly limited, and various host cells that have heretofore been used in a genetic engineering approach, such as *Escherichia coli, Bacillus subtilis,* yeast, filamentous fungi, and a plant or animal cell, may each be used. For example, when animal cells are used as the host cells, the HGF protein may be obtained by: subjecting the animal cells, such as a Chinese hamster ovary (CHO) cell, a mouse C127 cell, or a monkey COS cell, to transformation with an expression vector having incorporated thereinto cDNA encoding the amino acid sequence of a human HGF protein; separating the culture supernatant of the transformant; and purifying the supernatant through the above-mentioned column chromatography or the like.

The HGF protein thus obtained may have an amino acid sequence, which is obtained by substituting, deleting, and/or inserting one or a plurality of [e.g., one to several (the term "several" has the same meaning as that described above, and means, for example, 1 to 8, preferably 1 to 6, more preferably 1 to 5, still more preferably 1 to 3, particularly preferably 1 or 2; the same holds true for the following)] amino acids of, from, and/or into the amino acid sequence of a natural HGF protein, as long as the protein has bioactivity as a HGF protein. The substitution is preferably conservative substitution. In addition, similarly, a sugar chain may be substituted, deleted, or inserted into, from, or into the HGF protein. Herein, with regard to the amino acid sequence, the phrase "deleting, substituting, and/or inserting one or a plurality of amino acids" means that such a number of (typically one to several) amino acids that the acids may be produced by a well-known technical method, such as a genetic engineering approach or a site-directed mutagenesis method, or naturally occur are, for example, deleted, substituted, and/or inserted. The HGF protein into, from, or into which the sugar chain has been substituted, deleted, or inserted refers to, for example, a HGF protein obtained by treating the sugar chain added to the natural HGF protein with an enzyme or the like to delete the sugar chain, a protein obtained by subjecting the amino acid sequence of a site to which the sugar chain is to be added in the natural HGF protein to mutation so that the sugar chain may not be added thereto, or a protein obtained by subjecting the amino acid sequence of the natural HGF protein to mutation so that the sugar chain may be added to a site different from its natural site to which the sugar chain is to be added.

When the HGF protein to be used in the present disclosure is applied to a human, the above-mentioned human-derived protein is suitably used. However, a HGF protein derived from a mammalian animal except a human (e.g., a monkey, a cow, a horse, a pig, a sheep, a dog, a cat, a rat, a mouse, a rabbit, a hamster, a guinea pig, or a chimpanzee) may be used. Examples of such HGF protein include, but not limited to, a HGF protein derived from a mouse (e.g., Accession No. AAB31855, NP_034557, BAA01065, or BAA01064), a HGF protein derived from a rat [e.g., Accession No. NP_58713], a HGF protein derived from a cow (e.g., Accession No. NP_001026921, XP874086, or BAD02475), a HGF protein derived from a cat (e.g., Accession No. NP_001009830, BAC10545, or BAB21499), a HGF protein derived from a dog (e.g., Accession No. NP_001002964 or BAC57560), and a HGF protein derived from a chimpanzee (e.g., Accession No. XP519174) each registered in the NCBI database or the like.

The C-terminal of the HGF protein to be used in the present disclosure may be any one of a carboxyl group (-COOH), a carboxylate [-COOM (M represents a metal)], an amide (-CONH₂), or an ester (-COOR). Herein, as the R in the ester, there may be used, for example: a C1-6 alkyl group, such as a methyl, ethyl, n-propyl, isopropyl, or n-butyl group; a C3-8 cycloalkyl group, such as a cyclopentyl or cyclohexyl group; a C6-12 aryl group, such as a phenyl or α-naphthyl group; a C7-14 aralkyl group, such as a phenyl-C1-2 alkyl group, such as a benzyl or phenethyl group, or an α-naphthyl-C1-2 alkyl group such as an α-naphthylmethyl group; or a C2-6 alkanoylmethyl group, such as an acetyloxymethyl or pivaloyloxymethyl group. In the case where the HGF protein to be used in the present disclosure has a carboxyl group or a carboxylate at a site except the C-terminal, a protein obtained by amidating or esterifying the carboxyl group or the carboxylate is also included in the HGF protein in the present disclosure. For example, the ester at the C-terminal described above is used as an ester in this case. Further, the HGF protein to be used in the present disclosure includes: a protein obtained by protecting the amino group of a methionine residue at an N-terminal in the above-mentioned protein with a protective group (e.g., a C1-6 acyl group such as a formyl group or a C2-6 alkanoyl group such as an acetyl group); a protein obtained by turning a glutamyl group, which has been produced by the cleavage of the N-terminal side of the above-mentioned protein in a living organism, into pyroglutamic acid; a protein obtained by protecting a reactive group (e.g., -OH, -SH, an amino group, an imidazolyl group, an indolyl group, or a guanidino group) on a side chain of an amino acid in a molecule of the above-mentioned protein with a proper protective group (e.g., a C1-6 acyl group, such as a formyl group or a C2-6 alkanoyl group such as an acetyl group); or a conjugated protein such as a so-called glycoprotein having bonded thereto a sugar chain.

The term "gene encoding the HGF protein" as used in the present disclosure refers to a gene that can express the above-mentioned HGF protein. DNA holding the gene encoding the HGF protein (hereinafter sometimes referred to as "DNA encoding the HGF protein") is described in, for example, Nature, 342, 440 (1989); JP 2777678 B2; Biochem. Biophys. Res. Commun., 1989, vol. 163, pp. 967-973; or Proc. Natl. Acad. Sci. U.S.A., 1991, vol. 88 (No. 16), pp. 7001-7005, and is preferably, for example, DNA encoding a human-derived HGF protein registered as Accession No. M69718, M73240, AC004960, AY246560, M29145, or M73240 in GenBank, EMBL, or DDBJ.

In addition, when the DNA encoding the HGF protein to be used in the present disclosure is applied to a human, the above-mentioned human-derived protein is suitably used. However, DNA encoding a HGF protein derived from a mammalian animal except a human (e.g., a monkey, a cow, a horse, a pig, a sheep, a dog, a cat, a rat, a mouse, a rabbit, a hamster, a guinea pig, or a chimpanzee) may be used.

Examples of such DNA encoding the HGF protein include, but not limited to, DNA encoding a HGF protein derived from a mouse (e.g., Accession No. S71816, NM_010427, D10213, or D10212), DNA encoding a HGF protein derived from a rat (e.g., Accession No. NM_017017), DNA encoding a HGF protein derived from a cow (e.g., Accession No. NM_001031751 or AB110822), DNA encoding a HGF protein derived from a cat (e.g., Accession No. NM_001009830, AB080187, or AB046610), DNA encoding a HGF protein derived from a dog (e.g., Accession No. NM_001002964 or AB090353), and DNA encoding a HGF protein derived from a chimpanzee (e.g., Accession No. XM_519174) each registered in the NCBI database or the like.

Further, a preferred specific example of the DNA encoding the HGF protein is DNA having a base sequence represented by SEQ ID NO: 1 or 2. Herein, the base sequence represented by SEQ ID NO: 1 corresponds to a base sequence positioned at the 73rd to 2,259th positions of the base sequence of Accession No. M60718, and DNA formed of the base sequence corresponds to DNA encoding a HGF protein formed of an amino acid sequence represented by SEQ ID NO: 3. In addition, in a DNA recombination technology, when the HGF protein (SEQ ID NO: 3) expressed and produced in a cell is secreted to the outside of the cells, its signal sequence is cleaved to provide a HGF protein formed of an amino acid sequence represented by SEQ ID NO: 5. Accordingly, the DNA formed of the base sequence represented by SEQ ID NO: 1 corresponds to DNA encoding (producing) the HGF protein formed of the amino acid sequence represented by SEQ ID NO: 5. The base sequence represented by SEQ ID NO: 2 corresponds to a base sequence positioned at the 66th to 2,237th positions of the base sequence of Accession No. M73240, and DNA formed of the base sequence corresponds to DNA encoding a HGF protein formed of an amino acid sequence represented by SEQ ID NO: 4. For such HGF protein (SEQ ID NO: 4) as well, in the DNA recombination technology, when the protein is secreted to the outside of the cells, its signal sequence is cleaved to provide a HGF protein formed of an amino acid sequence represented by SEQ ID NO: 6. Accordingly, the DNA formed of the base sequence represented by SEQ ID NO: 2 corresponds to DNA encoding (producing) the HGF protein formed of the amino acid sequence represented by SEQ ID NO: 6.

### (Substance having c-Met Phosphorylation Action comparable to that of HGF Protein and Gene encoding the Substance)

It has been known that a HGF protein, which is a substance having a c-Met phosphorylation action, induces the phosphorylation of a c-Met receptor to provide an anti-apoptosis action, an angiogenesis action, a morphogenesis action, a cell division-promoting action, an axon-elongating action, or the like (see Proc Jpn Acad Ser B Phys Biol Sci. 2010; 86(6): 588-610).

That is, a substance having a c-Met phosphorylation action comparable to that of the HGF protein may be used as an effective component for each of the agents of the present disclosure as in the HGF protein.

Examples of the substance having a c-Met phosphorylation action comparable to that of the HGF protein may include, but not particularly limited to, the following substances. Examples thereof may include: low-molecular-weight compounds, such as ANG-3777 (Angion Biomedica Corp.: a Hepatocyte Growth Factor (HGF) Mimetic) and ATH-1017 (Athira Pharma, Inc.); cyclic peptide dimers, such as aML5, aMD4, and aMD5; a HGF alternative peptide (product code: PG-001; a c-Met agonist; PeptiGrowth Inc.); an anti-Met antibody AGMB-101 (AgomAb Therapeutics: AGMB-101 is a full MET agonist); HGF molecular fragment variants, such as NK1 dimer and K1K1; a DNA aptamer and an RNA aptamer; Internalin (Listeria protein); and a peptide having activity substantially identical in quality to that of the HGF protein.

As the peptide having activity substantially identical in quality to that of the HGF protein (hereinafter sometimes abbreviated as "HGF partial peptide"), any partial peptide of the above-mentioned HGF protein, the partial peptide having activity substantially identical in quality to that of the HGF protein, may be used. In the present disclosure, with regard to the number of the amino acids of the HGF partial peptide, for example, a peptide containing at least about 20 or more, preferably about 50 or more, more preferably about 100 or more amino acid sequences out of the amino acid sequences for forming the above-mentioned HGF protein is preferred. Specifically, for example, a peptide represented by a sequence ranging from the N-terminal hairpin loop of the HGF of a human HGF amino acid sequence to the first Kringle domain thereof is preferred.

In the HGF partial peptide of the present disclosure, its C-terminal may be any one of a carboxyl group (-COOH), a carboxylate [-COOM], an amide (-CONH₂), or an ester (-COOR). Further, as in the above-mentioned HGF protein, the HGF partial peptide includes: a partial peptide obtained by protecting the amino group of a methionine residue at the N-terminal of the above-mentioned partial peptide with a protective group; a partial peptide obtained by turning Gln, which has been produced by the cleavage of the N-terminal side thereof in a living organism, into pyroglutamic acid; a partial peptide obtained by protecting a substituent on a side chain of an amino acid in a molecule thereof with a proper protective group; or a conjugated peptide such as a so-called glycopeptide having bonded thereto a sugar chain.

A gene encoding the substance having a c-Met phosphorylation action to be used in the present disclosure is, for example, a gene encoding the above-mentioned peptide having a c-Met phosphorylation action.

### (Method of supplying HGF Protein or Substance having c-Met Phosphorylation Action comparable to that of HGF Protein, or Gene encoding HGF Protein or Gene encoding Substance having c-Met Phosphorylation Action comparable to that of HGF Protein)

The HGF protein or the substance having a c-Met phosphorylation action comparable to that of the HGF protein, or the gene encoding the HGF protein or the gene encoding the substance having a c-Met phosphorylation action comparable to that of the HGF protein (hereinafter sometimes collectively referred to as "HGF protein or the like") is not particularly limited as long as the HGF protein or the like is supplied to a lesion site of a spinal cord by intrathecal administration (in particular, administration to a subarachnoid cavity) or intraspinal administration.

More specifically, when the HGF protein or the like is administered to a patient, the HGF protein or the like may have various preparation forms, such as a liquid preparation and a solid preparation. In general, however, an injection, a propellant, or the like is prepared by using the HGF protein alone or in combination with a carrier to be commonly used together therewith. The above-mentioned injection may be any one of an aqueous injection and an oily injection.

When the aqueous injection is prepared, the aqueous injection may be prepared in accordance with a known method, for example, by: appropriately adding a pharmaceutically acceptable additive, such as a tonicity agent (e.g., sodium chloride, potassium chloride, glycerin, mannitol, sorbitol, boric acid, borax, glucose, or propylene glycol), a buffering agent (e.g., a phosphate buffer, an acetate buffer, a borate buffer, a carbonate buffer, a citrate buffer, a Tris buffer, a glutamate buffer, or an epsilon-aminocaproic acid buffer), a preservative (e.g., methyl parahydroxybenzoate, ethyl parahydroxybenzoate, propyl parahydroxybenzoate, butyl parahydroxybenzoate, chlorobutanol, benzyl alcohol, benzalkonium chloride, sodium dehydroacetate, sodium edetate, boric acid, or borax), a thickener (e.g., hydroxyethylcellulose, hydroxypropylcellulose, polyvinyl alcohol, or polyethylene glycol), a stabilizer (e.g., sodium hydrogen sulfite, sodium thiosulfate, sodium edetate, sodium citrate, ascorbic acid, or dibutylhydroxytoluene), or a pH adjuster (e.g., hydrochloric acid, sodium hydroxide, phosphoric acid, or acetic acid) to an aqueous solvent (e.g., water for injection or purified water) to prepare a solution; dissolving a HGF protein or the like in the solution; then sterilizing the resultant by filtration with a filter or the like; and then filling the resultant into an aseptic container. In addition, a suitable solubilizer, such as an alcohol (e.g., ethanol), a polyalcohol (e.g., propylene glycol or polyethylene glycol), or a nonionic surfactant (e.g., polysorbate 80 or polyoxyethylene hydrogenated castor oil 50), may be used.

When the oily injection is prepared, for example, sesame oil or soybean oil is used as an oily solvent, and benzyl benzoate, benzyl alcohol, or the like may be used as a solubilizer. The prepared injection is typically loaded into an appropriate ampule or vial.

The content of the HGF protein in the injection is adjusted to typically from about 0.0002 w/v% to about 2.0 w/v%, preferably from about 0.001 w/v% to about 1.0 w/v%, more preferably from about 0.01 w/v% to about 0.5 w/v%. A liquid preparation such as the injection is desirably stored after its moisture has been removed by cryopreservation, freeze drying, or the like. A freeze-dried preparation is used by adding distilled water for injection or the like to the preparation at the time of its use to redissolve the preparation.

The propellant may be prepared by a usual practice in preparation. When the HGF protein or the like is produced as the propellant, in addition to the propellent, for example, the solvent, the preservative, the stabilizer, the tonicity agent, or the pH adjuster described above is used as an additive therefor. A liquefied gas propellant, a compressed gas, or the like is used as the propellant. Examples of the liquified gas propellant include a fluorinated hydrocarbon (e.g., a hydrochlorofluorocarbon, such as HCFC-22, HCFC-123, HCFC-134a, or HCFC-142), liquified petroleum, and dimethyl ether. Examples of the compressed gas include a soluble gas (e.g., a carbon dioxide gas or a nitrous oxide gas), and an insoluble gas (e.g., a nitrogen gas).

In addition, the HGF protein to be used in the present disclosure may be turned into a sustained release preparation (e.g., a depot preparation) together with a biodegradable polymer. Particularly when the HGF protein is turned into a depot preparation, such effects as described below can be expected: a reduction in number of times of administration, the persistence of an action, and the alleviation of a side effect. The sustained release preparation may be produced in accordance with a known method. The biodegradable polymer to be used in the sustained release preparation may be appropriately selected from known biodegradable polymers. Examples thereof include: polysaccharides, such as starch, dextran, hyaluronan (hyaluronic acid) or a salt thereof, and chitosan; proteins, such as atelocollagen, collagen, and gelatin; polyamino acids, such as polyglutamic acid, polylysine, polyleucine, polyalanine, and polymethionine; polyesters, such as polylactic acid, polyglycolic acid, a lactic acid-glycolic acid copolymer, polycaprolactone, poly-β-hydroxybutyric acid, polymalic acid, polyacid anhydride, and a fumaric acid-polyethylene glycol-vinylpyrrolidone copolymer; polyorthoesters; polyalkyl cyanoacrylic acids such as polymethyl-α-cyanoacrylic acid; and polycarbonates, such as polyethylene carbonate or polypropylene carbonate. Of those, polyesters are preferred, and a lactic acid-glycolic acid copolymer is more preferred. In the case where the lactic acid-glycolic acid copolymer is used, its composition ratio (lactic acid/glycolic acid) (mol%) varies depending on its sustained release period, but for example, when the sustained release period is from about 2 weeks to 3 months, preferably from about 2 weeks to 1 month, the ratio is from about 100/0 to 50/50. The weight-average molecular weight of the lactic acid-glycolic acid copolymer is generally from about 5,000 to 20,000. The lactic acid-glycolic acid copolymer may be produced in accordance with a known production method such as a production method described in JP 61-28521 A. Although a blending ratio between the biodegradable polymer and the HGF protein is not particularly limited, the ratio of the HGF protein to the biodegradable polymer is, for example, from about 0.01 w/w% to about 30 w/w%.

An administration method is preferably as follows: the HGF protein is intrathecally administered (preferably administered into a subarachnoid cavity), is intraspinally administered, or is intrathecally and persistently administered with a sustained release pump, or the injection or the propellant is directly injected, infused, or supplied to a tissue having spinal cord injury. In addition, although a dose is appropriately selected in accordance with, for example, a dosage form, the degree of a disease, or an age, the dose is typically from 1 µg to 500 mg, preferably from 10 µg to 50 mg per administration. In addition, the administration method is also appropriately selected in accordance with, for example, the dosage form, the degree of the disease, or the age, and the HGF protein may be entirely administered once, or may be persistently administered for from about 30 minutes to about several weeks per administration (preferably for from about 24 hours to about 2 weeks per administration), or continuous administration may be performed by performing the single administration or the persistent administration at intervals. In the case of the continuous administration, an administration interval may be from once per day to once per several months. In the case of, for example, local (e.g., intrathecal) and persistent administration with a sustained release pump (e.g., an osmotic pump), the interval may be once per from several weeks to several months. Such persistent administration has an advantage in that the HGF protein is gradually released to a lesion site of a spinal cord over a long time period, and hence a HGF action is exhibited over a long time period to provide a better therapeutic effect. In addition, the administration has an advantage in that the number of times of administration is small, and hence a burden on a patient is alleviated. In addition, it can be said that the following point is an advantage: the HGF protein can be additionally administered as required to a subcutaneous osmotic pump that has been placed once. In addition, an administration timing is also appropriately selected in accordance with, for example, the dosage form, the degree of the disease, or the age, and is preferably within 14 days immediately after the traumatism of the spinal cord, more preferably within 7 days immediately after the traumatism, particularly preferably within 4 days immediately after the traumatism. Particularly in a patient of the spinal cord injury, in consideration of the fact that his or her condition is hardly stabilized for about 72 hours after the traumatism, the HGF protein is particularly preferably administered within from about 72 hours to about 4 days after the traumatism. The above-mentioned administration timing includes the time point at which the administration is started or the time point at which the administration is performed for the first time in the case of the persistent administration or the continuous administration.

For example, when the HGF protein is administered to a human, the following may be given as examples of an administration place and the like, but the place and the like are not particularly limited thereto:
administration place: administration from a lumbar vertebra into a subarachnoid cavity;
timing at which administration is started: immediately after injury, within 72 hours after the injury, or within from 48 hours to 72 hours after the injury;
number of times of administration: single administration is performed a plurality of times (from once to ten times, from twice to eight times, from three times to seven times, from four times to six times, or five times) every 1 day to 10 days (in particular, 7 days);
timing at which administration is ended: preferably from about 3 weeks to about 10 weeks, from 4 weeks to 8 weeks, or 5 weeks after the injury corresponding to a timing after the completion of the above-mentioned number of times of administration, though the timing varies depending on the degree of the injury and the degree of healing; and
HGF protein dose: from 100 µg/per administration to 2,000 µg/per administration, from 200 µg/per administration to 1,000 µg/per administration, from 300 µg/per administration to 700 µg/per administration, or about 400 µg/per administration.

### (Pluripotent Stem Cells)

Although the pluripotent stem cells of the present disclosure is not particularly limited as long as the cells can exhibit the effects of the agents of the present disclosure, examples of the pluripotent stem cells include: a differentiated cell-derived pluripotent stem cells (see JP 2009-215191 A) obtained by forcibly expressing each of an Oct3/4 gene, a Sox2 gene, and a Klf4 gene with a differentiated cell; a pluripotent stem cell (see JP 5603282 B2); an embryonic stem cell (ES cell); and an induced pluripotent stem cell (iPS cell).

A neural stem cell means a cell having a self-renewal ability and an ability to differentiate into a neural progenitor cell (neural stem progenitor cell). In addition, the neural progenitor cells refer to a cell, which is undifferentiated but is differentiated from the neural stem cells by one stage, and which performs the self-reproduction of itself to finally differentiate into a nerve cell. The neural stem cells or the neural progenitor cells are preferably obtained by the induction of differentiation from the pluripotent stem cells.

A method of inducing the differentiation of the pluripotent stem cells into the neural stem cells or the neural progenitor cells are not particularly limited, and a method to be typically performed may be adopted. For example, a neurosphere may be obtained by subjecting the pluripotent stem cells to suspension culture in a culture medium containing at least one kind of a bFGF or an EGF. In addition, the following may be repeated a plurality of times: the resultant neurosphere is dissociated into single cells; and the cells are subjected to suspension culture in a culture medium containing the bFGF again to form a neurosphere again.

For example, a human induced pluripotent cell line, such as a 201B7 cell, a 201B7-Ff cell, a 253G1 cell, a 253G4 cell, a 1201C1 cell, a 1205D1 cell, a 1210B2 cell, or a 1231A3 cell, is available (see WO 2021/045217 A1).

An iPS cell-derived neural stem cell and/or neural progenitor cells may be preferably, for example, a neural stem cell and/or neural progenitor cells derived from a human umbilical cord blood-derived human iPS cell (YZWJs513 hiPSC).

### (Method of transplanting Pluripotent Stem Cells)

Examples of a method of transplanting and administering the pluripotent stem cells include, but not particularly limited to, transplantation and injection. For example, the following method may be given as an example: the pluripotent stem cells are transplanted and administered to one or a plurality of places of a lesion site of a spinal cord. In addition, the cells may be administered by intravenous injection or the like to be caused to reach the lesion site of the spinal cord.

In addition, a γ-secretase inhibitor (GSI) is preferably added to clinical grade iPS cell-derived neural progenitor cells (YZWJs513 hiPSC-NS/PCs) on the day before the cell transplantation.

For example, when the pluripotent stem cells are transplanted to a human, the following may be given as examples of the timing at which the transplantation is started and the like:
timing at which transplantation is started: from about a second week to about a tenth week, or from a fourth week to a sixth week after injury;
number of times of transplantation: from once to five times, from once to three times, once or twice, or once; and
cell transplantation amount: from 100,000 cells/per transplantation to 50,000,000 cells/per transplantation, from 300,000 cells/per transplantation to 10,000,000 cells/per transplantation, from 500,000 cells/per transplantation to 5,000,000 cells/per transplantation, from 1,000,000 cells/per transplantation to 3,000,000 cells/per transplantation, or about 2,000,000 cells/per transplantation.

### (Therapeutic Agent for Spinal Cord Injury from Acute Phase to Subacute Phase)

The therapeutic agent for spinal cord injury from an acute phase to a subacute phase of the present disclosure includes the following:
(1) (a) a HGF protein or a substance having a c-Met phosphorylation action comparable to that of the HGF protein, or (b) a gene encoding the HGF protein or a gene encoding the substance having a c-Met phosphorylation action comparable to that of the HGF protein; and
(2) a pluripotent stem cell.

In addition, another aspect of the therapeutic agent for spinal cord injury from an acute phase to a subacute phase of the present disclosure includes the following:

### (1) the HGF protein.

The HGF protein is supplied to a lesion site of a spinal cord by intrathecal administration, and an iPS cell-derived neural stem cell and/or neural progenitor cells are administered to the lesion site of the spinal cord.

In addition, another aspect of the therapeutic agent for spinal cord injury from an acute phase to a subacute phase of the present disclosure includes the following:

### (1) the iPS cell-derived neural stem cell and/or neural progenitor cells.

The iPS cell-derived neural stem cell and/or neural progenitor cells are administered to the lesion site of the spinal cord during the supply of the HGF protein to the lesion site of the spinal cord by intrathecal administration, simultaneously with the supply, or after the supply.

### (Therapeutic Method for Spinal Cord Injury from Acute Phase to Subacute Phase)

A therapeutic method for spinal cord injury from an acute phase to a subacute phase of the present disclosure includes the following steps:
(1) a step of supplying (a) a hepatocyte growth factor protein or a substance having a c-Met phosphorylation action comparable to that of the hepatocyte growth factor protein, or (b) a gene encoding the hepatocyte growth factor protein or a gene encoding the substance having a c-Met phosphorylation action comparable to that of the hepatocyte growth factor protein to a lesion site of a spinal cord by intrathecal administration or intraspinal administration; and
(2) a step of administering a pluripotent stem cell to the lesion site of the spinal cord.

### (Kit for treating Spinal Cord Injury from Acute Phase to Subacute Phase)

A kit for treating spinal cord injury from an acute phase to a subacute phase of the present disclosure includes the following:
(1) (a) a hepatocyte growth factor protein or a substance having a c-Met phosphorylation action comparable to that of the hepatocyte growth factor protein, or (b) a gene encoding the hepatocyte growth factor protein or a gene encoding the substance having a c-Met phosphorylation action comparable to that of the hepatocyte growth factor protein; and
(2) a pluripotent stem cell.

The hepatocyte growth factor protein or the substance having a c-Met phosphorylation action comparable to that of the hepatocyte growth factor protein, or the gene encoding the hepatocyte growth factor protein or the gene encoding the substance having a c-Met phosphorylation action comparable to that of the hepatocyte growth factor protein is supplied to a lesion site of a spinal cord by intrathecal administration or intraspinal administration, and the pluripotent stem cells are administered to the lesion site of the spinal cord.

As a preferred aspect of each of the therapeutic agent for spinal cord injury from an acute phase to a subacute phase of the present disclosure, the therapeutic method for spinal cord injury from an acute phase to a subacute phase thereof, and the kit for treating spinal cord injury from an acute phase to a subacute phase thereof, the substance having a c-Met phosphorylation action is a HGF protein, the pluripotent stem cells are an iPS cell-derived neural stem cell and/or neural progenitor cells, and the spinal cord injury is severe spinal cord injury.

Further, as a preferred aspect of each of the therapeutic agent for spinal cord injury from an acute phase to a subacute phase of the present disclosure, the therapeutic method for spinal cord injury from an acute phase to a subacute phase thereof, and the kit for treating spinal cord injury from an acute phase to a subacute phase thereof, the HGF protein is supplied to the lesion site of the spinal cord by the intrathecal administration, and the iPS cell-derived neural stem cell and/or neural progenitor cells are administered to the lesion site.

Examples of treatment by each of the therapeutic agent for spinal cord injury from an acute phase to a subacute phase of the present disclosure, the therapeutic method for spinal cord injury from an acute phase to a subacute phase thereof, and the kit for treating spinal cord injury from an acute phase to a subacute phase thereof include the promotion of the regeneration of an injured spinal cord and/or the alleviation of an accessory symptom accompanying the spinal cord injury.

Examples of the promotion of the regeneration of the injured spinal cord include the promotion of axon extension, the inhibition of the atrophy of the injured spinal cord, an increase in survival and engraftment rate of pluripotent stem cells, the inhibition of demyelination after the spinal cord injury, an improvement in remyelination after the spinal cord injury, and/or the inhibition of cavitation in the injured spinal cord.

The promotion of the regeneration of the injured spinal cord in the present disclosure includes the activation of an endogenous nerve cell, the reconstruction of a neural network, and/or the promotion or encouragement of an increase in number of functional nerve fibers.

In addition, the promotion of the regeneration of the injured spinal cord in the present disclosure includes the reinforcement of neurotransmission by which recovery from the decline and attenuation of the neurotransmission due to the abnormality, rupture, or the like of the neurotransmission is achieved.

Further, the promotion of the regeneration of the injured spinal cord in the present disclosure includes the adjustment of an immunoreaction, the inhibition of inflammation, and/or the promotion or encouragement of angiogenesis.

Examples of the accessory symptom accompanying the injured spinal cord include motor dysfunction.

Examples of the motor dysfunction include lower limb motor dysfunction and motor dysfunction due to walking ability loss.

In the present disclosure, the walking includes gait.

### (Motor Dysfunction-alleviating Agent for Spinal Cord Injury from Acute Phase to Subacute Phase)

A motor dysfunction-alleviating agent for spinal cord injury from an acute phase to a subacute phase of the present disclosure includes the following:
(1) a HGF protein; and
(2) an iPS cell-derived neural stem cell and/or neural progenitor cell.

The HGF protein is supplied to a lesion site of a spinal cord by intrathecal administration, and the iPS cell-derived neural stem cells and/or neural progenitor cells are administered to the lesion site of the spinal cord.

A motor function is, for example, a lower limb motor function or a walking ability.

### (Inhibitor for Spinal Cord Cavitation in Spinal Cord Injury from Acute Phase to Subacute Phase)

An inhibitor for spinal cord cavitation in spinal cord injury from an acute phase to a subacute phase of the present disclosure includes the following:
(1) a HGF protein and a carrier, which carries, and is capable of sustained-releasing, the HGF protein; and
(2) an iPS cell-derived neural stem cell and/or neural progenitor cell.

The HGF protein is supplied to a lesion site of a spinal cord by intrathecal administration, and the iPS cell-derived neural stem cells and/or neural progenitor cells are administered to the lesion site of the spinal cord.

### (Inhibitor for Atrophy of Spinal Cord in Spinal Cord Injury from Acute Phase to Subacute Phase)

An inhibitor for atrophy of a spinal cord in spinal cord injury from an acute phase to a subacute phase of the present disclosure includes the following:
(1) a HGF protein and a carrier, which carries, and is capable of sustained-releasing, the HGF protein; and
(2) an iPS cell-derived neural stem cell and/or neural progenitor cell.

The HGF protein is supplied to a lesion site of a spinal cord by intrathecal administration, and the iPS cell-derived neural stem cells and/or neural progenitor cells are administered to the lesion site of the spinal cord.

### (Promoter for Axon Elongation in Lesion Site in Spinal Cord Injury from Acute Phase to Subacute Phase)

A promoter for axon elongation in a lesion site in spinal cord injury from an acute phase to a subacute phase of the present disclosure includes the following:
(1) a HGF protein and a carrier, which carries, and is capable of sustained-releasing, the HGF protein; and
(2) an iPS cell-derived neural stem cell and/or neural progenitor cell.

The HGF protein is supplied to a lesion site of a spinal cord by intrathecal administration, and the iPS cell-derived neural stem cells and/or neural progenitor cells are administered to the lesion site of the spinal cord.

### (Method of increasing Survival and Engraftment Rate of Pluripotent Stem Cells for Transplantation)

A method of increasing the survival and engraftment rate of pluripotent stem cells for transplantation of the present disclosure includes at least the following step:
(1) a step of bringing a hepatocyte growth factor into contact with the pluripotent stem cells for transplantation.

It has been recognized from Example 7 below that the survival and engraftment rate of the pluripotent stem cells for transplantation was increased by bringing the hepatocyte growth factor into contact with the pluripotent stem cells for transplantation.

The above-mentioned contact includes causing the hepatocyte growth factor to meet the pluripotent stem cells for transplantation in the same physical space (preferably a transplantation site) so that the factor may interact with at least part of the cells.

The following may be given as examples of preferred combinations in the agents of the present disclosure, the therapeutic agent thereof, and the production of the therapeutic agent, but the combinations are not particularly limited thereto:
(1) the hepatocyte growth factor protein or the substance having a c-Met phosphorylation action comparable to that of the hepatocyte growth factor protein, and the pluripotent stem cells;
(2) the gene encoding the hepatocyte growth factor protein or the gene encoding the substance having a c-Met phosphorylation action comparable to that of the hepatocyte growth factor protein, and the pluripotent stem cells;
(3) the hepatocyte growth factor protein or the substance having a c-Met phosphorylation action comparable to that of the hepatocyte growth factor protein, and the iPS cell-derived neural stem cells;
(4) the gene encoding the hepatocyte growth factor protein or the gene encoding the substance having a c-Met phosphorylation action comparable to that of the hepatocyte growth factor protein, and the iPS cell-derived neural stem cells;
(5) the hepatocyte growth factor protein, and the iPS cell-derived neural stem cells and/or neural progenitor cells.

In addition, the pluripotent stem cells of the item (1) may be combined with the hepatocyte growth factor protein of the item (5) to provide a combination of the hepatocyte growth factor protein and the pluripotent stem cells. Accordingly, the combinations of the items (1) to (5) are not particularly limited.

The present disclosure is described in detail below by way of specific examples. However, the present disclosure is not limited to these Examples.

All animal experiments were performed in accordance with the guideline of The Keio University Institutional Animal Care and Use Committee (approval number of 7,207 of Keio University).

### Example 1

### (Material and Method)

### (1) Human HGF Recombinant Protein

A human HGF recombinant protein formed of an amino acid sequence represented by SEQ ID NO: 6 was used. The protein was produced by using a CHO cell in accordance with a method described in Biochem. Biophys. Res. Commun. 180: 1151-1158, 1991.

### (2) Production of Spinal Cord Injury Model

An osmotic pump was aseptically prepared. The HGF protein (concentration: 1 mg/mL, dissolved in PBS) or the PBS was filled into an Alzet miniosmotic pump (manufactured by ALZA Corporation, Model 2002). A silicone tube having an inner diameter of 0.3 mm and an outer diameter of 0.7 mm (manufactured by Imamura; catheter tube) whose inner cavity was filled with the HGF protein or the PBS was connected to the ejecting portion of the pump, and the connecting portion was further covered with a silicone tube having an inner diameter of 1.0 mm and an outer diameter of 2.0 mm (manufactured by Imamura). The resultant was incubated at 37°C for 12 hours, and was then subjected to an experiment.

An adult female SD rat (having an age of about 8 weeks) was anesthetized by the intraperitoneal administration of 14 w/v% chloral hydrate, and the vertebral arches of its tenth thoracic vertebra and twelfth thoracic vertebra were removed. After that, the osmotic pump (filled with the HGF protein solution in advance by the above-mentioned method) was caused to indwell under its skin on a right spinal dorsal side, and the catheter tube was passed through its muscle layer from under the skin to be guided to the vertebral arch of the twelfth thoracic vertebra.

Next, severe contusion injury was produced in the tenth thoracic cord of the rat with an IH impactor (manufactured by Precision Systems, Inc.) at 240 kDyne, and then the dura and arachnoid of the twelfth thoracic cord thereof were split toward the head and buttock sides thereof. The catheter tube was inserted into the subarachnoid cavity thereof, and the tip of the catheter was advanced up to a position directly above the injured spinal cord. After that, the muscle layer and the skin were sutured to each other. Thus, an operation was completed.

### (3) Administration of HGF Protein

After the above-mentioned operation (corresponding to an acute phase after the contusion injury), the HGF protein solution was intrathecally administered with the osmotic pump over 2 weeks (dose of the human HGF recombinant protein: 200 µg/2 weeks). Only the PBS was administered to a control group.

### (4) Transplantation of iPS Cell-derived Neural progenitor Cells

A γ-secretase inhibitor (DAPT) was administered to clinical grade iPS cell-derived neural progenitor cells (YZWJs513 hiPSC-NS/PCs) on the day before cell transplantation. The clinical grade iPS cell-derived neural progenitor cells (YZWJs513 hiPSC-NS/PCs) (1×10⁶ cells) were transplanted to one point of a lesion epicenter on a ninth day after the injury (corresponding to a subacute phase after the contusion injury).

After that, the motor evaluation of the hindlimb motor function of the rat was performed with a Basso-Beattie-Bresnahan (BBB) score every other week. The motor function was observed for a time period of up to 84 days after the injury, and then the spinal cord thereof was collected.

### (5) Respective Groups

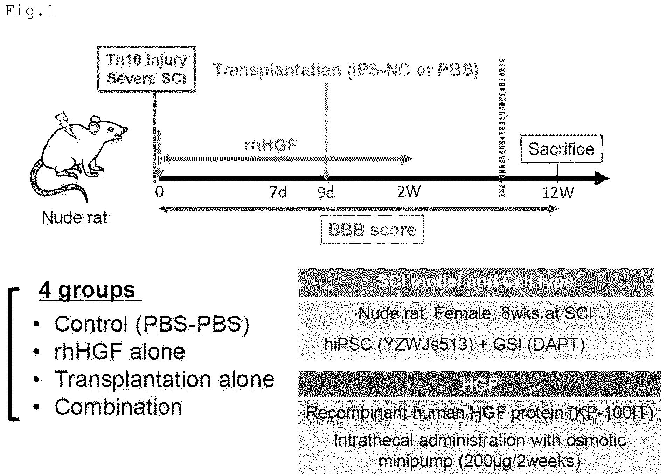

The respective groups were produced by the above-mentioned (1) to (4) (see FIG. 1).
Combination group: administration of human HGF recombinant protein+transplantation of iPS cell-derived neural progenitor cells
HGF alone group: human HGF recombinant protein+administration of PBS instead of transplantation of iPS cell-derived neural progenitor cells
TP alone group: administration of PBS instead of human HGF recombinant protein+transplantation of iPS cell-derived neural progenitor cells
Control group: administration of PBS instead of human HGF recombinant protein+administration of PBS instead of transplantation of iPS cell-derived neural progenitor cells

### (6) Evaluation Method

The tissue of the collected spinal cord was evaluated by methods based on immunostaining, myelin gold black staining, and an IVIS photon count using a fluorescent protein.

References for the respective evaluation methods were as follows: "Grafted human-induced pluripotent stem-cell-derived neurospheres promote motor functional recovery after spinal cord injury in mice, 2011;" "In vivo imaging of engrafted neural stem cells: its application in evaluating the optimal timing of transplantation for spinal cord injury, 2005;" and "High resolution neurochemical gold staining method for myelin in peripheral and central nervous system at the light- and electron-microscopic level, 2009."

### Example 2

### (Evaluation of Motor Function)

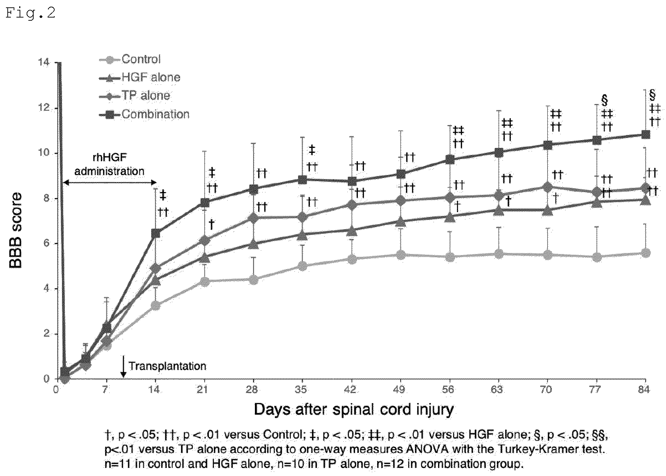

As the evaluation of a motor function (in particular, the evaluation of a lower limb motor function), a Basso-Beattie-Bresnahan (BBB, Basso et al., J. Neurotrauma, vol. 12, pp. 1-21, 1995) score and the stride length of a Digigait small animal gait analysis system were measured. The results are shown in FIG. 2 to FIG. 4.

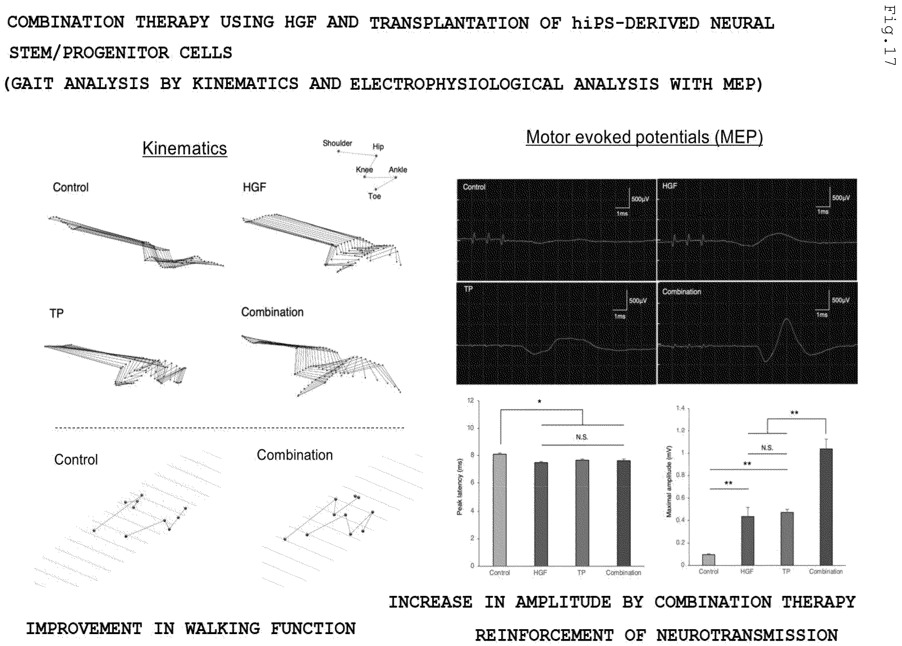

Further, gait analysis by kinematics analysis and the measurement of motor evoked potentials (MEPs) were performed. The results are shown in FIG. 17.

The BBB score of the combination group (group having administered thereto the agents of the present disclosure) gently increased until an 84th day, but those of the TP alone group and the HGF alone group did not increase until the 84th day (FIG. 2).

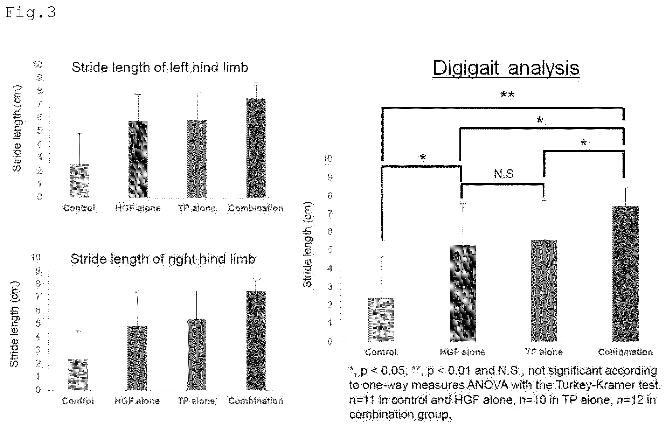

With regard to the lower limb stride length in the Digigait small animal gait analysis system, the value of the combination group significantly increased as compared to those of the TP alone group and the HGF alone group (FIG. 3).

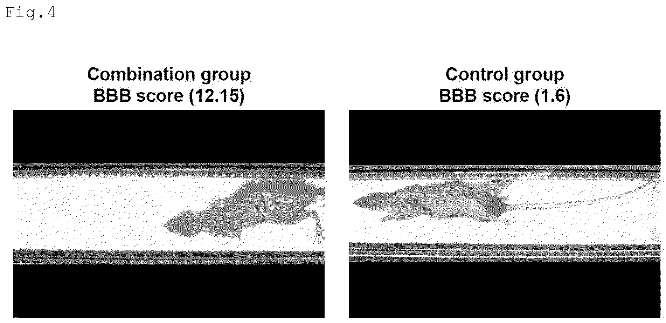

In addition, as shown in FIG. 4, in the combination group, a subacute-phase severe spinal cord injury model was able to acquire a walking ability. According to the conventional finding, in the HGF alone group (administration of the HGF protein), the subacute-phase severe spinal cord injury model was not able to acquire any walking ability.

Further, in the gait analysis by the kinematics analysis, in the combination group, the bending of a hindlimb joint enlarged, and hence an improvement in walking function was observed. In electrophysiological analysis with the MEPs, in the combination group, a large increase in amplitude of a motor evoked potential waveform was observed, and hence the reinforcement of neurotransmission was recognized (FIG. 17).

It is found from those results that each of the agents of the present disclosure has not only an improving effect on the motor function of a patient of spinal cord injury from an acute phase to a subacute phase but also the effect by which a patient of severe spinal cord injury is allowed to acquire a walking ability.

### Example 3

### (Histological Analysis and Evaluation)

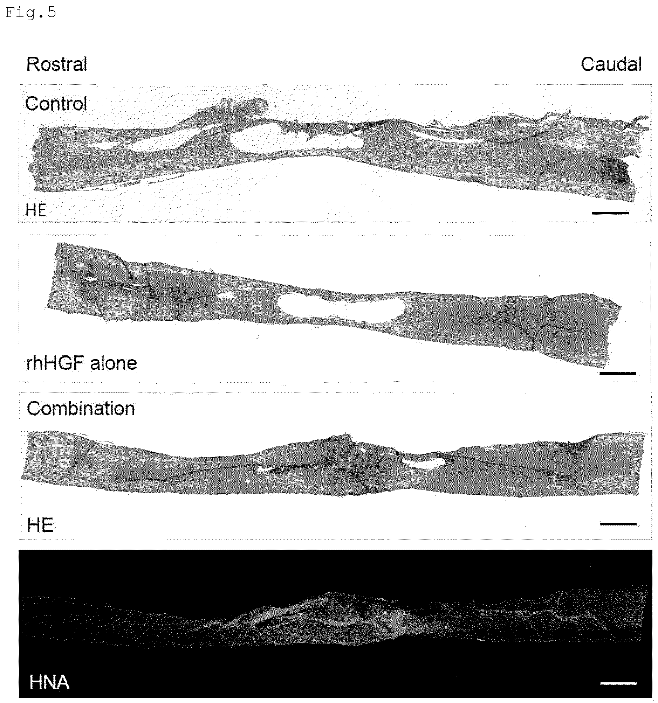

The result of the HE staining of the tissue of the collected spinal cord and the result of the human neutrophil alloantigen (HNA) fluorescent staining thereof are shown in FIG. 5. It was recognized that in the combination group (group having administered thereto the agents of the present disclosure), cavitation due to the contusion injury disappeared, and was replaced with a nerve cell as compared to the other groups.

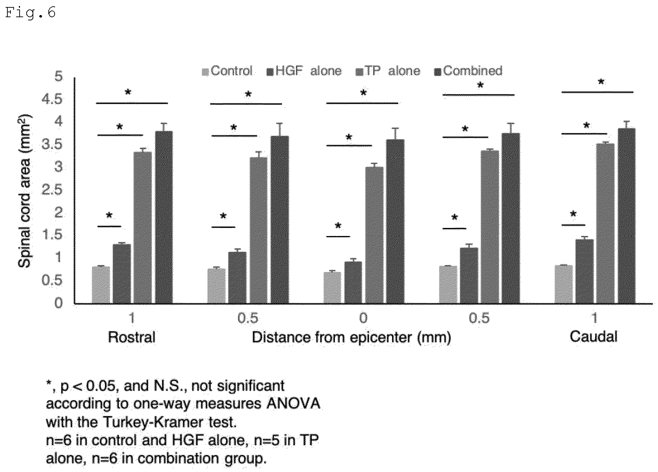

The result of the measurement of the area of a spinal cord area is shown in FIG. 6. It was recognized that the combination group had a spinal cord area wider than those of the other groups.

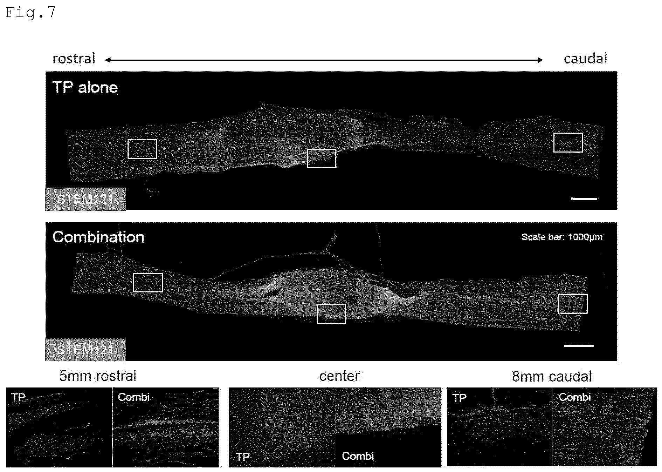

The result of the fluorescent staining of the tissue of the collected spinal cord with STEM121 (mouse monoclonal antibody specifically reacting with the intracytoplasmic protein of a human cell) is shown in FIG. 7. In the combination group (group having administered thereto the agents of the present disclosure), wide migration and axon elongation of the transplanted cells (iPS cell-derived neural progenitor cells derived from a human) toward the rostral and caudal regions of the rat were observed.

It is found from the foregoing that each of the agents of the present disclosure has an inhibiting effect on spinal cord cavitation, an inhibiting effect on the atrophy of an injured spinal cord, and a migration/axon elongation effect.

### Example 4

### (Evaluation of Differentiation of Transplanted Cells)

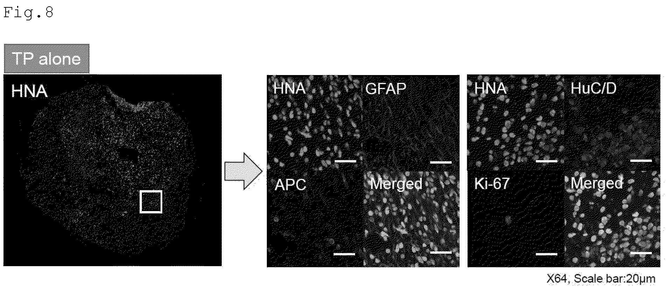

The survival of the iPS cell-derived neural progenitor cells was recognized by using HNA positive cells as a marker. Adenomatous polyposis coli (APC, mature oligodendrocyte marker) positive cells, glial fibrillary acidic protein (GFAP) positive cells, HuC/D (nerve cell-specific RNA-binding protein) positive cells, and Ki-67 positive cells were detected (FIG. 8).

It was recognized from the results of the detection that the iPS cell-derived neural progenitor cells derived from the human, which were transplanted cells, differentiated into three nervous systems (a neuron, an astrocyte, and an oligodendrocyte).

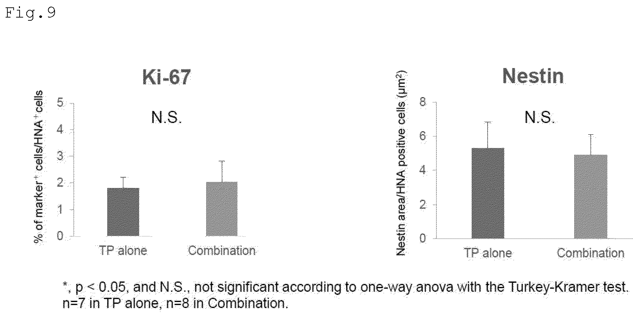

The measurement of the percentage of the Ki-67 positive cells (%) and the percentage of Nestin positive cells (%) found that in each of both the combination group and the TP alone group, no proliferation of proliferative cells and undifferentiated cells was observed, and hence a tumoral change was negative (FIG. 9).

Thus, it was recognized that the agents of the present disclosure were each free of an adverse event on a grafted cell (pluripotent stem cell) (in particular, the canceration of the grafted cell).

### Example 5

### (Evaluation of Regenerated Nerve Fibers and Serotonergic Neuron around Lesion Epicenter)

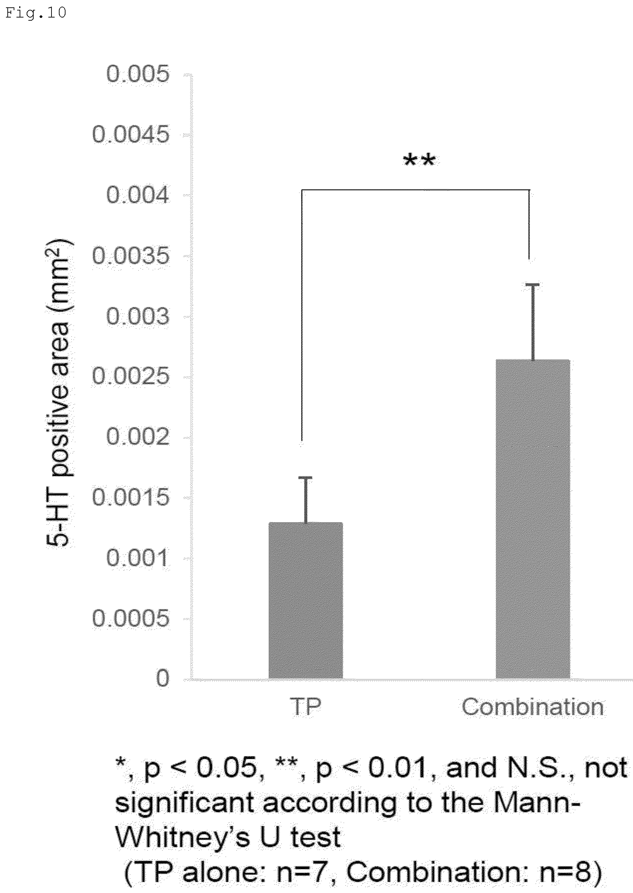

The amount of a serotonergic neuron (5-HT) playing an important role in the recovery of a lower limb motor function after spinal cord injury was measured. The results are shown in FIG. 10.

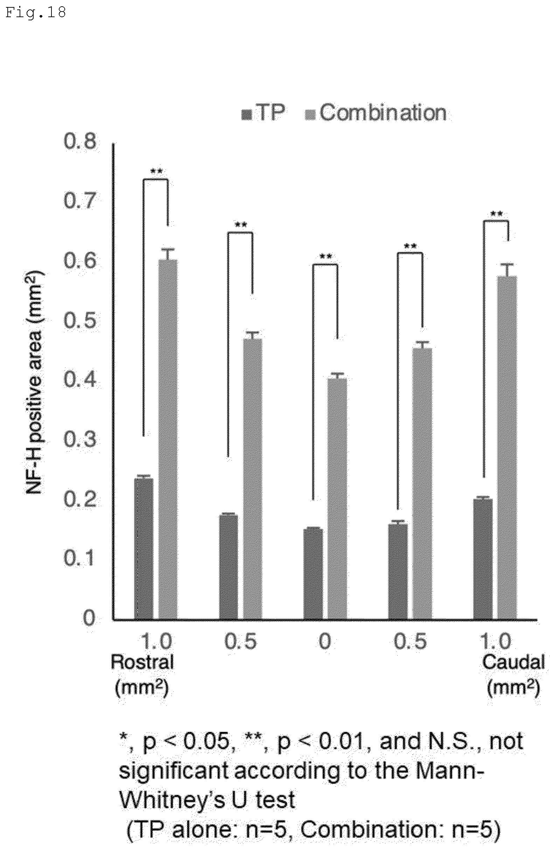

In addition, the amount of regenerated nerve fibers (NF-H) playing an important role in the recovery of a lower limb motor function after spinal cord injury was measured. The results are shown in FIG. 18.

In the combination group (group having administered thereto the agents of the present disclosure), cells that were 5-HT positive were observed over a range from the rostral region to the lesion epicenter. In addition, in the combination group, a larger number of 5-TH positive fibers were observed at a position caudal to a lesion site by 4 mm as compared to the TP alone group.

It was recognized that in the combination group (group having administered thereto the agents of the present disclosure), the number of the serotonergic neurons increased at a position (4 mm) caudal to the lesion epicenter as compared to that in the TP alone group.

In addition, in the combination group (group having administered thereto the agents of the present disclosure), there was observed a significant increase in number of NF-H positive nerve fibers at a position rostral or caudal to the lesion site by 1 mm as compared to the number thereof in the TP alone group.

Accordingly, the agents of the present disclosure each have a recovery effect on the lower limb motor function.

### Example 6

### (Quantitative Evaluation of Myelin Sheath Area around Lesion Epicenter)

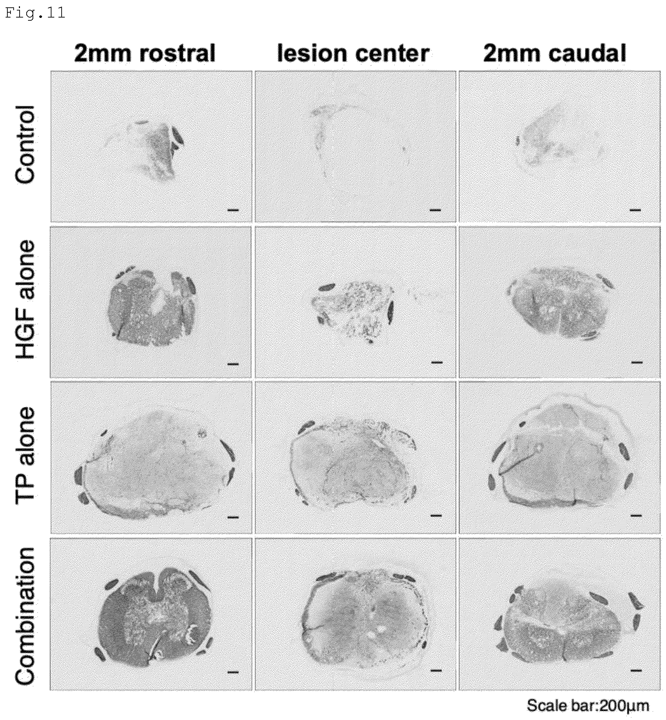

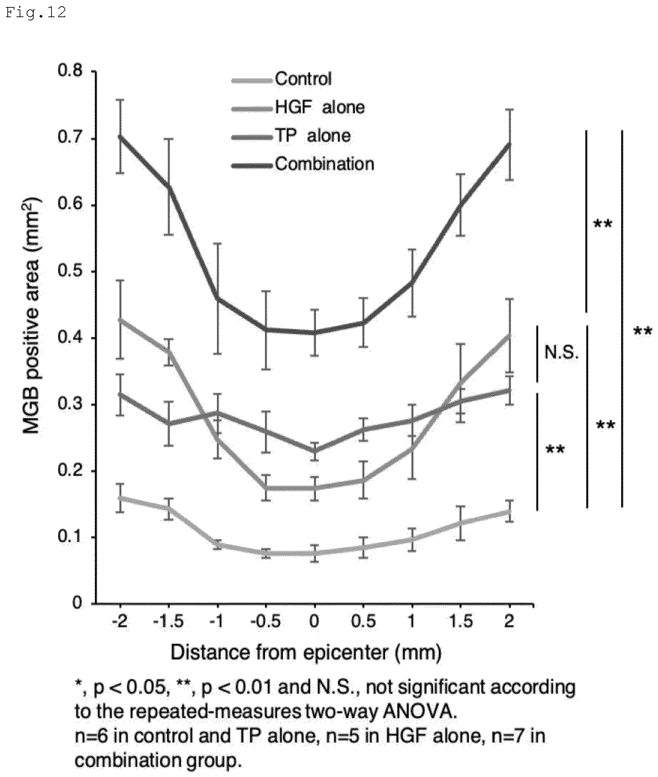

With regard to remyelination after spinal cord injury largely involved in the recovery of a lower limb motor function and an improvement in gait after the spinal cord injury, a myelin sheath area was quantitatively evaluated by using myelin gold black staining. The results are shown in FIG. 11 and FIG. 12.

In the control group, cavity formation and a demyelination finding around the lesion epicenter were observed. In the HGF alone group, a demyelination inhibition finding around the lesion epicenter was observed. In the TP alone group, a remyelination finding in the lesion epicenter was observed. In the combination group, there was observed a significant increase in myelin sheath area by demyelination inhibition and remyelination as compared to myelin sheath areas in the other groups.

It is found from the foregoing that each of the agents of the present disclosure has an inhibiting effect on demyelination after spinal cord injury and an improving effect on remyelination thereafter.

### Example 7

### (Evaluation of Survival and Engraftment Rate of Transplanted Cells)

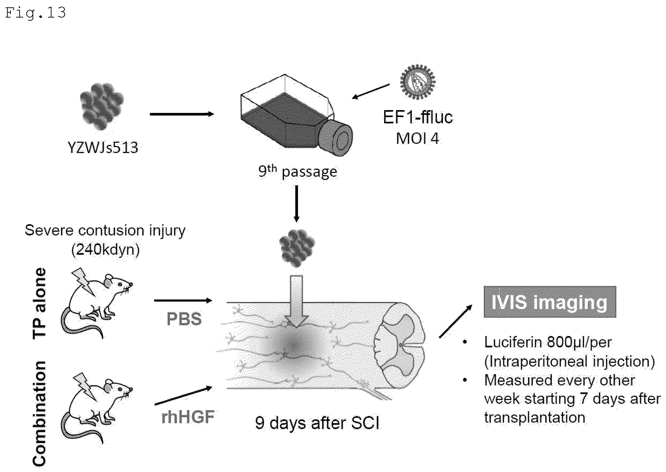

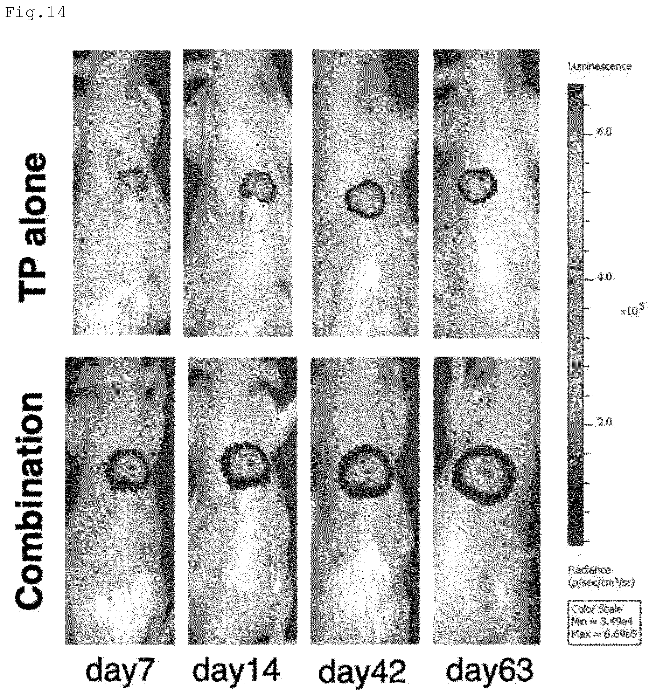

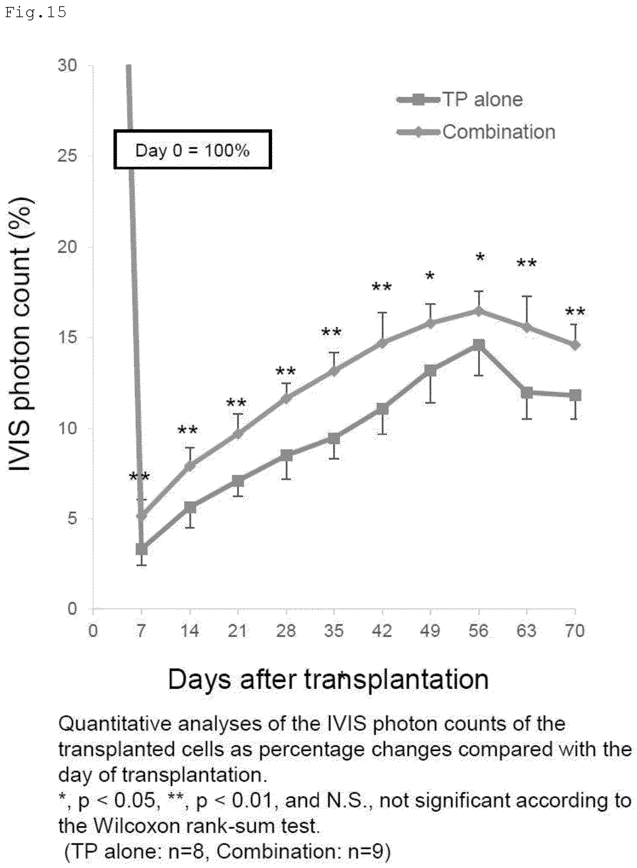

EF1-fffluc (fluorescent protein-fused luciferase: MOI4) was added to cells (YZWJs513), and the cells were subcultured (9^{th}). The cells (YZWJs513) after the subculture were transplanted to the spinal cord injury model rat (Example 1). After the transplantation, luciferin serving as a fluorescent protein was intraperitoneally administered (800 µg/per administration) thereto every other week, and the survival and engraftment rate of the transplanted cells was measured by IVIS imaging (FIG. 13). The measurement results are shown in FIG. 14 and FIG. 15.

It was recognized that in the combination group (group having administered thereto the agents of the present disclosure), the survival and engraftment rate of the transplanted cells increased as compared to that in the TP alone group.

It is found from the foregoing that each of the agents of the present disclosure has an increasing effect on the survival and engraftment rate of transplanted cells (pluripotent stem cells).

### Example 8

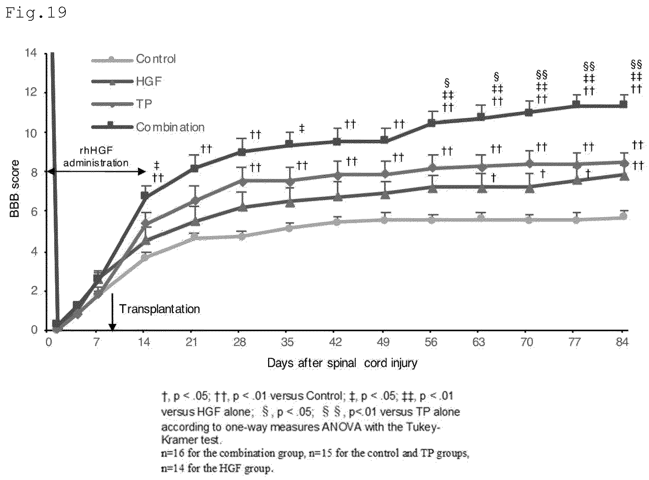

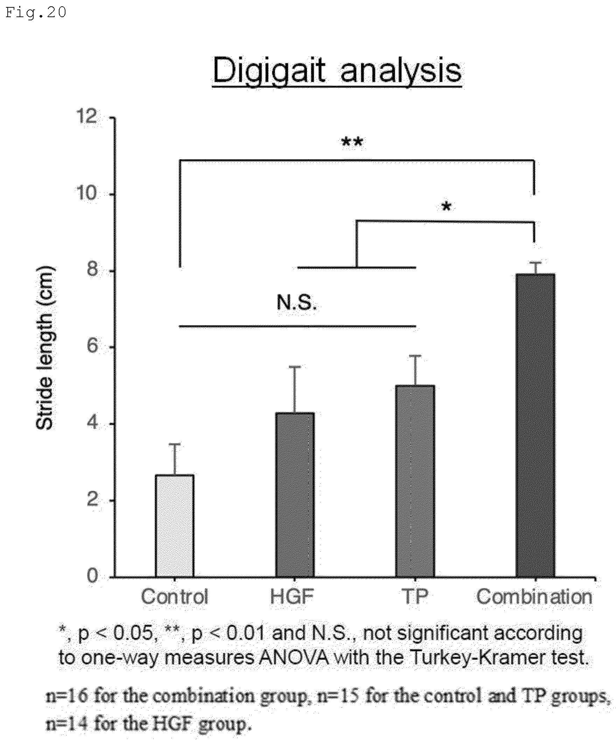

The results of the BBB score and the stride length of the Digigait small animal gait analysis system in the number of cases (combination group: 16, HGF alone group: 14, TP alone group: 15, control group: 15) increased from the number of the cases of Example 2 (combination group: 12, HGF alone group: 11, TP alone group: 10, control group: 11) are shown in FIG. 19 and FIG. 20, respectively.

### (General Remarks on Examples)

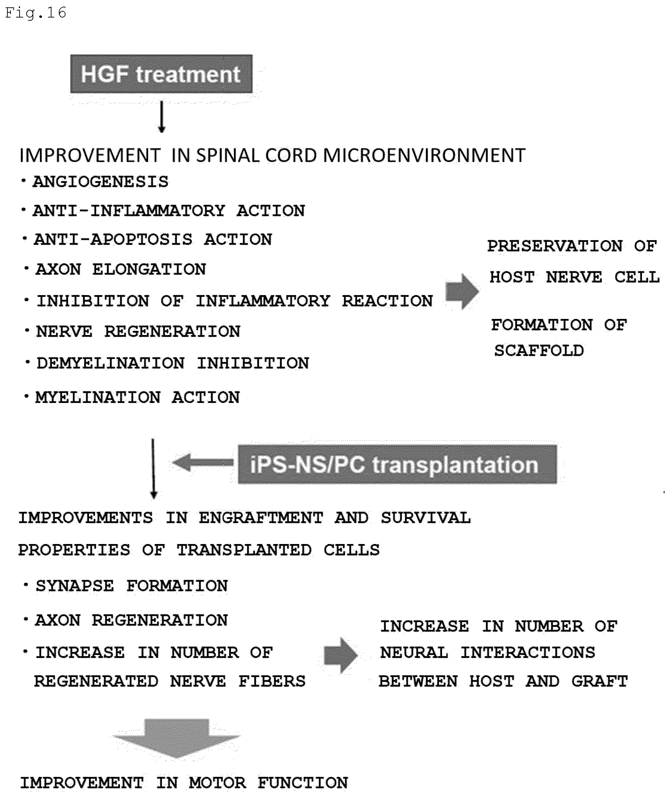

It is conceivable from the results of Examples 1 to 7 that the agents of the present disclosure each have the following effects on the basis of the mechanism illustrated in FIG. 16:
(1) an alleviating effect on motor dysfunction due to spinal cord injury (a recovery effect on a lower limb motor function or a walking ability-acquiring effect);
(2) an inhibiting effect on spinal cord cavitation in a lesion site of a spinal cord;
(3) an inhibiting effect on the atrophy of an injured spinal cord;
(4) a migration/axon elongation effect in the lesion site of the spinal cord;
(5) an increasing effect on the survival and engraftment rate of transplanted cells;
(6) an inhibiting effect on demyelination after the spinal cord injury;
(7) an improving effect on remyelination after the spinal cord injury; and
(8) a reinforcing effect on neurotransmission in the lesion site of the spinal cord.

In addition, a substance having a c-Met phosphorylation action comparable to that of the HGF protein may have the same action and effect as those of the HGF protein.

### Industrial Applicability

There can be provided a therapeutic agent for spinal cord injury from an acute phase to a subacute phase.

## Claims

1. A therapeutic agent for spinal cord injury from an acute phase to a subacute phase, comprising the following:
(1) (a) a hepatocyte growth factor protein or a substance having a c-Met phosphorylation action comparable to that of the hepatocyte growth factor protein, or (b) a gene encoding the hepatocyte growth factor protein or a gene encoding the substance having a c-Met phosphorylation action comparable to that of the hepatocyte growth factor protein; and
(2) a pluripotent stem cell.

2. The therapeutic agent according to claim 1, wherein the pluripotent stem cells are an iPS cell-derived neural stem cell and/or neural progenitor cells.

3. The therapeutic agent according to claim 1, wherein the spinal cord injury is severe spinal cord injury.

4. The therapeutic agent according to claim 1, wherein the substance having a c-Met phosphorylation action is a hepatocyte growth factor protein, the pluripotent stem cells are an iPS cell-derived neural stem cell and/or neural progenitor cells, and the spinal cord injury from the acute phase to the subacute phase is severe spinal cord injury.

5. The therapeutic agent according to claim 4, wherein the hepatocyte growth factor protein is intrathecally administered.

6. The therapeutic agent according to claim 5, wherein the hepatocyte growth factor protein is administered into a subarachnoid cavity.

7. The therapeutic agent according to any one of claims 1 to 6, wherein the treatment is promotion of regeneration of an injured spinal cord and/or alleviation of an accessory symptom accompanying the spinal cord injury.

8. The therapeutic agent according to claim 7, wherein the promotion of the regeneration of the injured spinal cord is promotion of axon extension, inhibition of atrophy of the spinal cord, an increase in survival and engraftment rate of the pluripotent stem cells, inhibition of demyelination, an improvement in remyelination, and/or inhibition of spinal cord cavitation.

9. The therapeutic agent according to claim 7, wherein the promotion of the regeneration of the injured spinal cord is activation of an endogenous nerve cell, reconstruction of a neural network, and/or promotion or encouragement of an increase in number of functional nerve fibers.

10. The therapeutic agent according to claim 7, wherein the accessory symptom accompanying the injured spinal cord is motor dysfunction.

11. The therapeutic agent according to claim 10, wherein the motor dysfunction is motor dysfunction of a lower limb.

12. The therapeutic agent according to claim 11, wherein the motor dysfunction is walking ability loss.

13. A therapeutic agent for spinal cord injury from an acute phase to a subacute phase, comprising a hepatocyte growth factor protein,
wherein the hepatocyte growth factor protein is supplied to a lesion site of a spinal cord by intrathecal administration, and an iPS cell-derived neural stem cell and/or neural progenitor cells are administered to the lesion site of the spinal cord.

14. A therapeutic agent for spinal cord injury from an acute phase to a subacute phase, comprising an iPS cell-derived neural stem cell and/or neural progenitor cells,
wherein the iPS cell-derived neural stem cell and/or neural progenitor cells are administered to a lesion site of a spinal cord during or after supply of a hepatocyte growth factor protein to the lesion site of the spinal cord by intrathecal administration.

15. A motor dysfunction-alleviating agent for spinal cord injury from an acute phase to a subacute phase, comprising the following:
(1) a hepatocyte growth factor protein; and
(2) an iPS cell-derived neural stem cell and/or neural progenitor cells,
wherein the hepatocyte growth factor protein is supplied to a lesion site of a spinal cord by intrathecal administration, and the iPS cell-derived neural stem cell and/or neural progenitor cells are administered to the lesion site of the spinal cord.

16. The motor dysfunction-alleviating agent according to claim 15, wherein the motor function is a lower limb motor function or a walking function.

17. An inhibitor for demyelination after spinal cord injury for spinal cord injury from an acute phase to a subacute phase, comprising the following:
(1) a hepatocyte growth factor protein; and
(2) an iPS cell-derived neural stem cell and/or neural progenitor cells,
wherein the hepatocyte growth factor protein is supplied to a lesion site of a spinal cord by intrathecal administration, and the iPS cell-derived neural stem cell and/or neural progenitor cells are administered to the lesion site of the spinal cord.

18. An improver for remyelination after spinal cord injury for spinal cord injury from an acute phase to a subacute phase, comprising the following:
(1) a hepatocyte growth factor protein; and
(2) an iPS cell-derived neural stem cell and/or neural progenitor cells,
wherein the hepatocyte growth factor protein is supplied to a lesion site of a spinal cord by intrathecal administration, and the iPS cell-derived neural stem cell and/or neural progenitor cells are administered to the lesion site of the spinal cord.

19. An inhibitor for spinal cord cavitation for spinal cord injury from an acute phase to a subacute phase, comprising the following:
(1) a hepatocyte growth factor protein; and
(2) an iPS cell-derived neural stem cell and/or neural progenitor cells,
wherein the hepatocyte growth factor protein is supplied to a lesion site of a spinal cord by intrathecal administration, and the iPS cell-derived neural stem cell and/or neural progenitor cells are administered to the lesion site of the spinal cord.

20. An inhibitor for atrophy of a spinal cord for spinal cord injury from an acute phase to a subacute phase, comprising the following:
(1) a hepatocyte growth factor protein; and
(2) an iPS cell-derived neural stem cell and/or neural progenitor cells,
wherein the hepatocyte growth factor protein is supplied to a lesion site of a spinal cord by intrathecal administration, and the iPS cell-derived neural stem cell and/or neural progenitor cells are administered to the lesion site of the spinal cord.

21. A promoter for axon elongation in a lesion site for spinal cord injury from an acute phase to a subacute phase, comprising the following:
(1) a hepatocyte growth factor protein; and
(2) an iPS cell-derived neural stem cell and/or neural progenitor cells,
wherein the hepatocyte growth factor protein is supplied to a lesion site of a spinal cord by intrathecal administration, and the iPS cell-derived neural stem cell and/or neural progenitor cells are administered to the lesion site of the spinal cord.

22. A promoter for reconstruction of a neural network of a lesion site and/or an increase in number of functional never fibers thereof for spinal cord injury from an acute phase to a subacute phase, comprising the following:
(1) a hepatocyte growth factor protein; and
(2) an iPS cell-derived neural stem cell and/or neural progenitor cells,
wherein the hepatocyte growth factor protein is supplied to a lesion site of a spinal cord by intrathecal administration, and the iPS cell-derived neural stem cell and/or neural progenitor cells are administered to the lesion site of the spinal cord.

23. A therapeutic method for spinal cord injury from an acute phase to a subacute phase, comprising the following steps:
(1) a step of supplying (a) a hepatocyte growth factor protein or a substance having a c-Met phosphorylation action comparable to that of the hepatocyte growth factor protein, or (b) a gene encoding the hepatocyte growth factor protein or a gene encoding the substance having a c-Met phosphorylation action comparable to that of the hepatocyte growth factor protein to a lesion site of a spinal cord by intrathecal administration or intraspinal administration; and
(2) a step of administering a pluripotent stem cell to the lesion site of the spinal cord.

24. A kit for treating spinal cord injury from an acute phase to a subacute phase, comprising the following:
(1) (a) a hepatocyte growth factor protein or a substance having a c-Met phosphorylation action comparable to that of the hepatocyte growth factor protein, or (b) a gene encoding the hepatocyte growth factor protein or a gene encoding the substance having a c-Met phosphorylation action comparable to that of the hepatocyte growth factor protein; and
(2) a pluripotent stem cell,
wherein the hepatocyte growth factor protein or the substance having a c-Met phosphorylation action comparable to that of the hepatocyte growth factor protein, or the gene encoding the hepatocyte growth factor protein or the gene encoding the substance having a c-Met phosphorylation action comparable to that of the hepatocyte growth factor protein is supplied to a lesion site of a spinal cord by intrathecal administration or intraspinal administration, and the pluripotent stem cells are administered to the lesion site of the spinal cord.

25. A method of increasing a survival and engraftment rate of pluripotent stem cells for transplantation, comprising bringing a hepatocyte growth factor into contact with the pluripotent stem cells for transplantation.
